(19) Europäisches Patentamt
European Patent Office
Office européen des brevets

(11) EP 3 991 731 A1

(12) **EUROPEAN PATENT APPLICATION**
published in accordance with Art. 153(4) EPC

(43) Date of publication:
**04.05.2022 Bulletin 2022/18**

(21) Application number: **20832137.2**

(22) Date of filing: **28.06.2020**

(51) International Patent Classification (IPC):
*A61K 31/495* (2006.01) *C07D 471/04* (2006.01)
*A61P 35/00* (2006.01) *A61P 35/02* (2006.01)

(52) Cooperative Patent Classification (CPC):
**A61K 31/495; A61P 35/00; A61P 35/02; C07D 471/04**

(86) International application number:
**PCT/CN2020/098474**

(87) International publication number:
**WO 2020/259679 (30.12.2020 Gazette 2020/53)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.06.2019 CN 201910577816**
**15.04.2020 CN 202010296869**
**28.05.2020 CN 202010466633**

(71) Applicant: **Tuojie Biotech (Shanghai) Co., Ltd.**
**Shanghai 201203 (CN)**

(72) Inventors:
- **ZOU, Hao**
**Shanghai 201203 (CN)**
- **LI, Zhengtao**
**Shanghai 201203 (CN)**
- **WANG, Yuanhao**
**Shanghai 201203 (CN)**
- **YU, Jian**
**Shanghai 201203 (CN)**
- **ZHU, Wei**
**Shanghai 201203 (CN)**

(74) Representative: **Potter Clarkson**
**The Belgrave Centre**
**Talbot Street**
**Nottingham NG1 5GG (GB)**

(54) **PYRIMIDINE FIVE-MEMBERED NITROGEN HETEROCYCLIC DERIVATIVE, PREPARATION METHOD THEREOF AND PHARMACEUTICAL USE THEREOF**

(57) Disclosed are a pyrimidine five-membered nitrogen heterocyclic derivative, a preparation method thereof and the pharmaceutical use thereof. Specifically disclosed are a pyrimidine five-membered nitrogen heterocyclic derivative represented by general formula (II), a preparation method thereof, a composition containing the derivative, and the use thereof as an SHP2 inhibitor and in the preparation of a medicament for preventing and/or treating tumors or cancer.

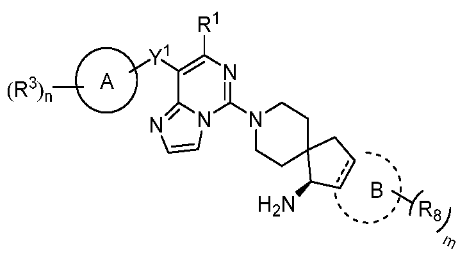

(II)

EP 3 991 731 A1

## Description

### TECHNICAL FIELD

The present disclosure belongs to the field of medicine and relates to a pyrimidine fused five-membered nitrogen-containing heterocycle derivative, a preparation method thereof and the pharmaceutical use thereof. Specifically, the present disclosure relates to a pyrimidine fused five-membered nitrogen-containing heterocycle derivative of general formula (I), a preparation method thereof, a composition containing the derivative, and a use thereof as an SHP2 inhibitor and in the preparation of a medicament for preventing and/or treating tumor or cancer.

### BACKGROUND

Src homology domain 2 containing tyrosine phosphatase-2 (SHP2) is an evolutionarily conserved non-receptor protein tyrosine phosphatase (PTP) encoded by the PTPN11 gene. It mainly consists of two SH2 domains (N-SH2, C-SH2) and one PTP catalytic domain. It is widely expressed in various human tissues and plays an important role in maintaining tissue development and cell homeostasis, and the like. SHP2 is related to signaling through Ras-mitogen-activated protein kinase, JAK-STAT or phosphoinositide 3-kinase AKT pathway. Mutations in the PTPN11 gene and subsequent mutations in SHP2 have been identified in a variety of human diseases, for example Noonan syndrome, Leopard syndrome, juvenile myelomonocytic leukemia, neuroblastoma, melanoma, acute myeloid leukemia, as well as breast cancer, lung cancer and colon cancer (same as Claim 19). Therefore, SHP2 represents a highly attractive target for the development of new therapies for treating various diseases.

Published patent applications of research related to SHP2 target include WO2018136264A, WO2015003094A, WO2018160731A, WO2018130928A1, WO2018136265A, WO2018172984A, WO2018081091, WO2016203405, WO2017211303A, WO2018013597A, and the like. Currently, the SHP2 inhibitor TNO155 developed by Novartis and the SHP2 inhibitor SHP2 JAB-3068 developed by JACOBIO are both in the phase I clinical trial, and there is no marketed product for this target. Therefore, it is still necessary to continue to develop new SHP2 inhibitors with higher effcacy in order to provide patients with new and effective anti-cancer drugs.

### SUMMARY OF THE INVENTION

The present disclosure provides a compound of general formula (I) or a tautomer, mesomer, racemate, enantiomer, diastereomer, atropisomer thereof, or mixture form thereof, or a pharmaceutically acceptable salt thereof, characterized in that

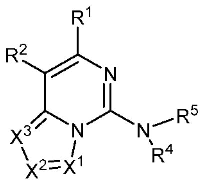

(I)

$R^1$ is selected from the group consisting of hydrogen atom, deuterium atom, hydroxy, cyano, nitro, halogen, carboxy, alkyl, alkoxy, haloalkyl, haloalkoxy, amino, alkenyl and hydroxyalkyl; $R^2$ is selected from

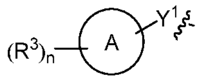

, wherein $Y^1$ is selected from the group consisting of -S-, -NH-, $-S(O)_2-$, $-S(O)_2-NH-$, $-C(=CH_2)-$, -S(O)- and direct bond;

ring A is selected from the group consisting of cycloalkyl, heterocycloalkyl, aryl and heteroaryl, the cycloalkyl, heterocycloalkyl, aryl and heteroaryl are each a 5-12 membered monocycle or polycycle;

each $R^3$ is independently selected from the group consisting of hydrogen atom, deuterium atom, halogen, cyano, amino, nitro, carboxy, hydroxy, hydroxyalkyl, $C_{3-8}$ cycloalkyl, $C_{3-10}$ heterocyclyl, aryl, heteroaryl, $C_{2-6}$ alkenyl, $C_{4-8}$ cycloalkenyl, $C_{2-6}$ alkynyl, $-NR^aR^b$, $-alkenyl-NR^aR^b$, $-alkenyl-O-R^a$, $-alkenyl-C(O)_2R^a$, $-alkenyl-R^a$, $-alkenyl-CO-NR^aR^b$, $-alkenyl-NR^a-CO-NR^aR^b$, $-alkenyl-NR^a-C(O)R^b$, $-C(O)NR^aR^b$, $-C(O)R^a$, $-CO-alkenyl-NR^aR^b$, $-NR^aC(O)R^b$,

-$C(O)_2R^a$, -O-alkenyl-CO-$OR^a$, -O-alkenyl-CO-$NR^aR^b$, -O-alkenyl-$NR^aR^b$, -$OR^a$, -$SR^a$ -$NR^a$-CO-$NR^aR^b$, -$NR^a$-alkenyl-$NR^aR^b$, -$NR^a$-alkenyl-$R^b$, -$NR^aS(O)2R^b$, -$NR^aS(O)R^b$, -$NR^aS(O)2NR^aR^b$, -$NR^aS(O)NR^aR^b$, -$S(O)_2NR^aR^b$, -$S(O)NR^aR^b$, -$S(O)R^a$, -$S(O)_2R^a$, -$P(O)R^aR^b$, -$N(S(O)R^aR^b)$ and -$S(O)(NR^a)R^b$, the aryl or heteroaryl is optionally further substituted by one or more selected from the group consisting of halogen, hydrogen atom, deuterium atom, cyano, amino, nitro, carboxy, hydroxy, hydroxyalkyl, alkyl, alkoxy, haloalkyl and haloalkoxy;

the $R^a$ and $R^b$ are each independently selected from the group consisting of hydrogen, deuterium atom, halogen, amino, hydroxy, cyano, nitro, carboxy, alkyl, alkoxy, haloalkyl, haloalkoxy, $C_{5-10}$ heteroaryl and aryl, the aryl or heteroaryl is optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy;

n is selected from the group consisting of 0, 1, 2, 3, 4 and 5;

$X^1$, $X^2$ and $X^3$ are each independently selected from the group consisting of $CR^c$ and N, and wherein at least one of them is N; the $R^c$ is selected from the group consisting of hydrogen atom, deuterium atom, $C_{1-6}$ alkyl, $C_{1-6}$ alkoxy, $C_{1-6}$ alkylthio, amino, nitro, hydroxy, carbonyl, carboxy, halogen and cyano, preferably $X^1$ is $CR^c$;

$R^4$ is selected from the group consisting of hydrogen, $C_{1-6}$ alkyl, 3-12 membered monocyclic heterocyclic ring or polycyclic heterocyclic ring and $C_{3-8}$ cycloalkyl, the each alkyl, heterocyclyl or cycloalkyl is optionally substituted by one or more groups selected from the group consisting of halogen, hydroxy, $C_{1-3}$ alkyl, amino, alkylamino, hydroxyalkyl and alkoxy;

$R^5$ is selected from the group consisting of hydrogen, hydroxy, $C_{1-6}$ alkyl and $C_{3-8}$ cycloalkyl, the alkyl or cycloalkyl is optionally substituted by one or more aminos; or

$R^4$ and $R^5$ together with the nitrogen atom to which they are attached form a 3-12 membered monocyclic heterocyclic ring or polycyclic heterocyclic ring, the each monocyclic heterocyclic ring or polycyclic heterocyclic ring is optionally substituted by one or more groups selected from the group consisting of halogen, hydroxy, halogen-substituted or unsubstituted $C_{1-6}$ alkyl, amino, alkylamino, hydroxyalkyl, heteroaryl, heterocyclyl, alkylamino, and halogen-substituted or unsubstituted alkoxy, the polycyclic heterocyclic ring includes, but is not limited to, bridged heterocyclic ring and spirocyclic heterocyclic ring;

exemplary rings formed by $R^4$ and $R^5$ together with the nitrogen atom to which they are attached include, but are not limited to:

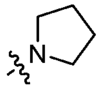 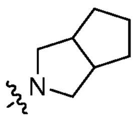 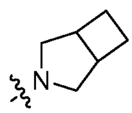 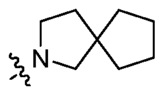

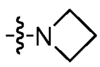 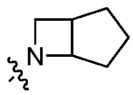 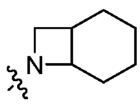 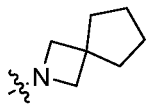 ;

or $R^4$ and $R^5$ together with the nitrogen atom to which they are attached form

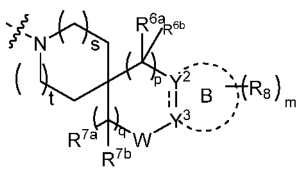

wherein s and t are optionally selected from the group consisting of 0 and 1;

each $R^{6a}$ or $R^{6b}$ is independently selected from the group consisting of hydrogen atom, deuterium atom, fluorine atom, amino, hydroxy, cyano, nitro, carboxy, fluorine-substituted or unsubstituted alkyl and fluorine-substituted or unsubstituted alkoxy; or $R^{6a}$ and $R^{6b}$ together with the carbon atom to which they are attached form CO, C=NH, C=N-OH, 3-12 membered heterocyclyl or $C_{3-8}$ cycloalkyl;

p is selected from the group consisting of 0, 1, 2, 3 and 4;

each R7a or R7b is independently selected from the group consisting of hydrogen atom, deuterium atom, fluorine atom, amino, hydroxy, cyano, nitro, carboxy, fluorine-substituted or unsubstituted alkyl, fluorine-substituted or unsubstituted alkoxy and -$NR^aS(O)NR^aR^b$;

or $R^{7a}$ and $R^{7b}$ together with the carbon atom to which they are attached form a 3-10 membered heterocyclyl, 5-10

membered heteroaryl, $C_{3-8}$ cycloalkyl or C=NR$^{7c}$, the $R^{7c}$ is selected from the group consisting of hydrogen atom, deuterium atom and $C_{1-6}$ alkyl, the ring is optionally substituted;
q is selected from the group consisting of 0, 1, 2, 3 and 4;
W is absent or is selected from the group consisting of -O, -S and -NR$^w$, the $R^w$ is selected from the group consisting of hydrogen atom, halogen, amino, hydroxy, cyano, nitro, carboxy, -C(O)$C_{1-6}$ alkyl, -C(O)$_2C_{1-6}$ alkyl, $C_{1-6}$ alkyl ether, halogen-substituted or unsubstituted $C_{1-6}$ alkyl and halogen-substituted or unsubstituted $C_{1-6}$ alkoxy;
ring B is absent or is a 3-10 membered ring;
$\overset{---}{---}$ is a single bond or double bond;
when ring B is absent, $Y^2$ is $CR^{2a}R^{2b}$, $NR^{2a}$ or O, $Y^3$ is $CR^{3a}R^{3b}$, $NR^{3a}$ or O;
when ring B is a 3-10 membered ring:

1) $Y^2$ is $CR^{2a}$ or N, $Y^3$ is $CR^{3a}$ or N, $\overset{---}{---}$ is a single bond; or
2) $Y^2$ is C and $Y^3$ is C, $\overset{---}{---}$ is a double bond;

each $R^{2a}$, $R^{2b}$, $R^{3a}$ or $R^{3b}$ is independently selected from the group consisting of hydrogen atom, deuterium atom, halogen, amino, hydroxy, cyano, nitro, carboxy, halogen-substituted or unsubstituted alkyl and halogen-substituted or unsubstituted alkoxy;
each $R^8$ is independently selected from the group consisting of hydrogen atom, deuterium atom, halogen, cyano, amino, nitro, carboxy, hydroxy, hydroxyalkyl, $C_{3-8}$ cycloalkyl, $C_{3-10}$ heterocyclyl, aryl, heteroaryl, $C_{2-6}$ alkenyl, $C_{4-8}$ cycloalkenyl, $C_{2-6}$ alkynyl, -NR$^a$R$^b$, -alkenyl-NR$^a$R$^b$, -alkenyl-O-R$^a$, -alkenyl-C(O)$_2$R, -alkenyl-R$^a$, -alkenyl-CO-NR$^a$R$^b$, -alkenyl-NR$^a$-CO-NR$^a$R$^b$, -alkenyl-NR$^a$-C(O)R$^b$, -C(O)NR$^a$R$^b$, -C(O)R$^a$, -CO-alkenyl-NR$^a$R$^b$, -NR$^a$C(O)R$^b$, -C(O)$_2$R$^a$, -O-alkenyl-CO-OR$^a$, -O-alkenyl-CO-NR$^a$R$^b$, -O-alkenyl-NR$^a$R$^b$, -OR$^a$, -SR$^a$ -NR$^a$-CO-NR$^a$R$^b$, -NR$^a$-alkenyl-NR$^a$R$^b$, -NR$^a$-alkenyl-R$^b$, -NR$^a$S(O)2R$^b$, -NR$^a$S(O)R$^b$, -NR$^a$S(O)2NR$^a$R$^b$, -NR$^a$S(O)NR$^a$R$^b$, -S(O)$_2$NR$^a$R$^b$, -S(O)NR$^a$R$^b$, -S(O)R$^a$, -S(O)$_2$R$^a$, -P(O)R$^a$R$^b$, -N(S(O)R$^a$R$^b$) and -S(O)(NR$^a$)R$^b$, the aryl or heteroaryl is optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy;
the $R^a$ and $R^b$ are each independently selected from the group consisting of hydrogen, deuterium atom, halogen, amino, hydroxy, cyano, nitro, carboxy, alkyl, alkoxy, haloalkyl, haloalkoxy, $C_{5-10}$ heteroaryl and aryl, the aryl or heteroaryl is optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy;
m is selected from the group consisting of 0, 1, 2, 3 and 4;
or two $R^8$ are attached together to form a 6-membered aromatic ring, 5-membered heteroaryl, 6-membered heteroaryl or $C_{3-6}$ heterocyclyl, the each ring is optionally substituted or unsubstituted, the substituent(s) is selected from the group consisting of halogen, amino, hydroxy, cyano, nitro and $C_{1-6}$ alkyl.
In some embodiments, the compound of general formula (I) or the tautomer, mesomer, racemate, enantiomer, diastereomer, atropisomer thereof, or mixture form thereof, or the pharmaceutically acceptable salt thereof according to the present disclosure is characterized in that the $R^1$ is selected from the group consisting of hydrogen atom, deuterium atom, $C_{1-6}$ alkyl, $C_{1-6}$ alkoxy, amino and hydroxy.

In some embodiments, the compound of general formula (I) or the tautomer, mesomer, racemate, enantiomer, diastereomer, atropisomer thereof, or mixture form thereof, or the pharmaceutically acceptable salt thereof according to the present disclosure is characterized in that:

$Y^1$ is selected from the group consisting of -S- and direct bond;
ring A is selected from the group consisting of aryl and heteroaryl;
each $R^3$ is independently selected from the group consisting of hydrogen atom, deuterium atom, halogen, $C_{1-6}$ alkyl, halo$C_{1-6}$ alkyl, halo$C_{1-6}$ alkoxy, $C_{1-6}$ alkoxy, cyano, amino, nitro, carboxy, hydroxy and phenyl, the phenyl is optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy; preferably hydrogen atom, deuterium atom, halogen, halo$C_{1-6}$ alkyl, $C_{1-6}$ alkyl, $C_{1-6}$ alkoxy, halo$C_{1-6}$ alkoxy and phenyl, the phenyl is optionally further substituted by one or more substituents selected from the group consisting of halogen, hydrogen atom, deuterium atom, cyano, amino, nitro, carboxy, hydroxy, hydroxyalkyl, alkyl, alkoxy, haloalkyl and haloalkoxy;
n is selected from the group consisting of 0, 1, 2, 3, 4 and 5.

In some embodiments, the compound of general formula (I) or the tautomer, mesomer, racemate, enantiomer, diastereomer, atropisomer thereof, or mixture form thereof, or the pharmaceutically acceptable salt thereof according

to the present disclosure is characterized in that the $X^1$, $X^2$ and $X^3$ are each independently selected from the group consisting of $CR^c$ and N, wherein at least one of them is N, and the $R^c$ is hydrogen atom.

In some preferred embodiments, the $X^1$ is $CR^c$, and the $R^c$ is hydrogen atom.

In some embodiments, the compound of general formula (I) or the tautomer, mesomer, racemate, enantiomer, diastereomer, atropisomer thereof, or mixture form thereof, or the pharmaceutically acceptable salt thereof according to the present disclosure is characterized in that the $R^4$, $R^5$ together with the nitrogen atom to which they are attached form

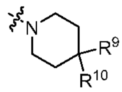 ,

wherein $R^9$ and $R^{10}$ are each independently selected from the group consisting of hydrogen atom, deuterium atom, hydroxy, $C_{1-6}$ alkyl, $C_{1-6}$ alkoxy, halogen, $C_{1-6}$ hydroxyalkyl, aryl, heteroaryl, heterocyclyl, amino, $C_{1-6}$ alkylamino and $-NR^aS(O)NR^aR^b$; or

$R^a$ and $R^b$ are as defined in general formula (I) in the claims.

In some preferred embodiments, $R^9$ and $R^{10}$ are each independently selected from the group consisting of hydrogen atom, deuterium atom, $C_{1-6}$ alkyl, amino and $-NR^aS(O)NR^aR^b$, $R^a$ and $R^b$ are as described in claim 1.

In some embodiments of the present disclosure, the compound of general formula (I) or the tautomer, mesomer, racemate, enantiomer, diastereomer, atropisomer thereof, or mixture form thereof, or the pharmaceutically acceptable salt thereof is characterized in that $R^4$, $R^5$ together with the nitrogen atom to which they are attached form

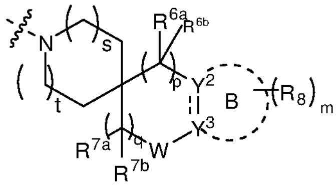

wherein s and t are optionally selected from the group consisting of 0 and 1;

- $R^{6a}$ and $R^{6b}$ are each independently selected from the group consisting of hydrogen atom, deuterium atom, $C_{1-6}$ alkyl and $C_{1-6}$ alkoxy; or $R^{6a}$ and $R^{6b}$ together with the carbon atom to which they are attached form a 3-12 membered heterocyclyl or $C_{3-8}$ cycloalkyl;
- p is selected from the group consisting of 0, 1 and 2;
- $R^{7a}$ and $R^{7b}$ are each independently selected from the group consisting of hydrogen atom, deuterium atom, amino, $C_{1-6}$ alkyl and $-NR^aS(O)NR^aR^b$, $R^a$ and $R^b$ are as described in claim 1;
- q is 1 or 2;
- W is absent;
- ring B is absent or is a 3-10 membered ring;
- $\stackrel{---}{—}$ is a single bond or double bond;
- when ring B is absent, $Y^2$ is $CR^{2a}R^{2b}$ or O, $Y^3$ is $CR^{3a}R^{3b}$; or
- when ring B is a 3-10 membered ring:
  - $Y^2$ is $CR^{2a}$ or N, $Y^3$ is $CR^{3a}$ or N, $\stackrel{---}{—}$ is a single bond; or
  - $Y^2$ is C and $Y^3$ is C, $\stackrel{---}{—}$ is a double bond;
  - each $R^{2a}$, $R^{2b}$ and $R^{3a}$ is independently selected from the group consisting of hydrogen atom, deuterium atom and $C_{1-6}$ alkyl;
  - each $R^8$ is independently selected from the group consisting of hydrogen atom, deuterium atom, halogen, amino, hydroxy, cyano, nitro, carboxy, $C_{1-6}$ alkyl and $C_{1-6}$ alkoxy;
  - m is selected from the group consisting of 0, 1, 2, 3 and 4; or two $R^8$ are attached together to form a 6-membered aromatic ring, 5-membered heteroaryl, 6-membered heteroaryl or $C_{3-6}$ heterocyclyl, the each ring is optionally substituted or unsubstituted, the substituent(s) is selected from the group consisting of halogen, amino, hydroxy, cyano, nitro and $C_{1-6}$ alkyl.

In some embodiments of the present disclosure, the

$R^1$ is selected from the group consisting of hydrogen atom, deuterium atom, methyl and amino;
$Y^1$ is selected from the group consisting of -S- and direct bond;
ring A is selected from the group consisting of aryl and heteroaryl;
each $R^3$ is independently selected from the group consisting of hydrogen atom, deuterium atom, halogen, halo$C_{1-6}$ alkyl, $C_{1-6}$ alkyl, $C_{1-6}$ alkoxy, halo$C_{1-6}$ alkoxy and substituted phenyl;
n is selected from the group consisting of 0, 1, 2, 3, 4 and 5;
$X^1$, $X^2$ and $X^3$ are each independently selected from the group consisting of $CR^c$ and N, wherein at least one of them is N, the $X^1$ is $CR^c$ and the $R^c$ is hydrogen atom;
$R^4$ and $R^5$ together with the nitrogen atom to which they are attached form

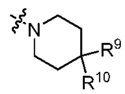 ,

$R^9$ and $R^{10}$ are each independently selected from the group consisting of hydrogen atom, deuterium atom, $C_{1-6}$ alkyl, amino and $-NR^aS(O)NR^aR^b$, $R^a$ and $R^b$ are as defined in general formula (I).

In some preferred embodiments of the present disclosure, the

$R^1$ is selected from the group consisting of hydrogen atom, deuterium atom, methyl and amino;
$Y^1$ is selected from the group consisting of -S- and direct bond;
ring A is selected from the group consisting of aryl and heteroaryl;
each $R^3$ is independently selected from the group consisting of hydrogen atom, deuterium atom, halogen, halo$C_{1-6}$ alkyl, $C_{1-6}$ alkyl, $C_{1-6}$ alkoxy, halo$C_{1-6}$ alkoxy and substituted phenyl;
n is selected from the group consisting of 0, 1, 2, 3, 4 and 5;
$X^1$, $X^2$ and $X^3$ are each independently selected from the group consisting of $CR^c$ and N, wherein at least one of them is N, the $X^1$ is $CR^c$ and the $R^c$ is hydrogen atom;
$R^{6a}$ and $R^{6b}$ are each independently selected from the group consisting of hydrogen atom, deuterium atom, $C_{1-6}$ alkyl and $C_{1-6}$ alkoxy; or $R^{6a}$ and $R^{6b}$ together with the carbon atom to which they are attached form a 3-12 membered heterocyclyl or $C_{3-8}$ cycloalkyl;
p is 1 or 2;
$R^{7a}$ and $R^{7b}$ are each independently selected from the group consisting of hydrogen atom, deuterium atom, amino, $C_{1-6}$ alkyl and $-NR^aS(O)NR^aR^b$, $R^a$ and $R^b$ are as defined in general formula (I);
q is 1 or 2;
W is absent;
ring B is absent, $Y^2$ is $CR^{2a}R^{2b}$ or O, $Y^3$ is $CR^{3a}R^{3b}$;
each $R^{2a}$, $R^{2b}$, $R^{3a}$ and $R^{3b}$ is independently selected from the group consisting of hydrogen atom, deuterium atom and $C_{1-6}$ alkyl.

In some preferred embodiments of the present disclosure, the

$R^1$ is selected from the group consisting of hydrogen atom, deuterium atom, methyl and amino;
$Y^1$ is selected from the group consisting of -S- and direct bond;
ring A is selected from the group consisting of aryl and heteroaryl;
each $R^3$ is independently selected from the group consisting of hydrogen atom, deuterium atom, halogen, halo$C_{1-6}$ alkyl, $C_{1-6}$ alkyl, $C_{1-6}$ alkoxy, halo$C_{1-6}$ alkoxy and substituted phenyl;
n is selected from the group consisting of 0, 1, 2, 3, 4 and 5;
$X^1$, $X^2$ and $X^3$ are each independently selected from the group consisting of $CR^c$ and N, wherein at least one of them is N, the $X^1$ is $CR^c$ and the $R^c$ is hydrogen atom;
$R^{6a}$ and $R^{6b}$ are each independently selected from the group consisting of hydrogen atom, deuterium atom, $C_{1-6}$ alkyl and $C_{1-6}$ alkoxy;
p is 1 or 2;
$R^{7a}$ and $R^{7b}$ are each independently selected from the group consisting of hydrogen atom, deuterium atom, amino, $C_{1-6}$ alkyl and $-NR^aS(O)NR^aR^b$, $R^a$ and $R^b$ are as defined in general formula (I);
q is 1 or 2;
W is absent;
ring B is a 6-membered aryl ring, 5-membered heteroaryl or 6-membered heteroaryl;

$Y^2$ is C and $Y^3$ is C, $\overline{- - -}$ is a double bond;
each $R^8$ is independently selected from the group consisting of hydrogen atom, deuterium atom, halogen, amino, hydroxy, cyano, nitro, carboxy, $C_{1-6}$ alkyl and $C_{1-6}$ alkoxy;
m is selected from the group consisting of 0, 1, 2, 3 and 4.

In alternative embodiments of the present disclosure,

$R^4$ and $R^5$ together with the nitrogen atom to which they are attached form

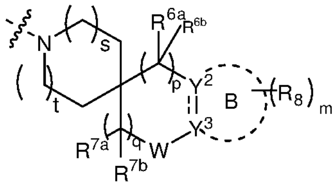

$R^1$ is selected from the group consisting of hydrogen atom and methyl;
$Y^1$ is -S-;
ring A is selected from the group consisting of aryl and heteroaryl;
each $R^3$ is independently selected from the group consisting of hydrogen atom, deuterium atom, halogen, amino, halo$C_{1-6}$ alkyl, $C_{1-6}$ alkyl, $C_{1-6}$ alkoxy, halo$C_{1-6}$ alkoxy and $C_{1-6}$ alkylamino;
n is selected from the group consisting of 0, 1, 2, 3, 4 and 5;
$X^3$ is N, the $X^1$ and $X^2$ are $CR^c$, and the $R^c$ is hydrogen atom;
s and t are optionally selected from the group consisting of 0 and 1;
$R^{6a}$ and $R^{6b}$ are each independently selected from the group consisting of hydrogen atom, deuterium atom, $C_{1-6}$ alkyl and $C_{1-6}$ alkoxy;
p is 1;
$R^{7a}$ and $R^{7b}$ are each independently selected from the group consisting of hydrogen atom, deuterium atom, amino and $C_{1-6}$ alkyl;
q is 1;
W is absent;
ring B is a 6-membered aromatic ring, 5-membered heteroaromatic ring or 6-membered heteroaromatic ring;
$Y^2$ is C and $Y^3$ is C, $\overline{- - -}$ is a double bond;
each $R^8$ is independently selected from the group consisting of hydrogen atom, deuterium atom, halogen, amino, hydroxy, cyano, nitro, carboxy, $C_{1-6}$ alkyl and $C_{1-6}$ alkoxy;
m is selected from the group consisting of 0, 1, 2, 3 and 4.

The present disclosure provides a compound of general formula (II) or a tautomer, mesomer, racemate, enantiomer, diastereomer, atropisomer thereof, or mixture form thereof, or a pharmaceutically acceptable salt thereof, wherein

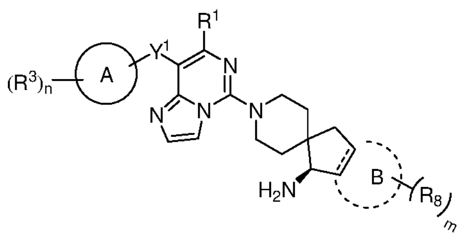

(II)

$R^1$ is selected from the group consisting of hydrogen atom, $C_{1-6}$ alkyl, halo$C_{1-6}$ alkyl and amino, the alkyl and haloalkyl are each independently optionally further substituted by one or more substituents of deuterium atom;

$Y^1$ is -S- or a direct bond;
ring A is selected from the group consisting of aryl and heteroaryl, preferably phenyl and pyridyl;
each $R^3$ is independently selected from the group consisting of hydrogen atom, deuterium atom, halogen, cyano,

$C_{1-6}$ alkyl, $C_{1-6}$ alkoxy, halo$C_{1-6}$ alkyl, halo$C_{1-6}$ alkoxy, $C_{3-8}$ cycloalkyl, 3-12 membered heterocyclyl, $-OR^a$, $-CHR^aR^b$ and $-NR^aR^b$;

the $R^a$ and $R^b$ are each independently selected from the group consisting of hydrogen, deuterium atom, hydroxy, $C_{1-6}$ alkyl, 3-12 membered heterocyclyl and $C_{3-8}$ cycloalkyl, wherein the alkyl, heterocyclyl or cycloalkyl is optionally further substituted by one or more substituents selected from the group consisting of halogen, deuterium atom, cyano, amino and hydroxy;

or $R^a$ and $R^b$ together with the atom to which they are attached form a 3-12 membered heterocyclyl or $C_{3-8}$ cycloalkyl, the alkyl, heterocyclyl or cycloalkyl is optionally further substituted by one or more substituents selected from the group consisting of halogen, deuterium atom, cyano, amino and hydroxy;

ring B is a 6-membered aromatic ring, 5-membered heteroaromatic ring or 6-membered heteroaromatic ring, preferably benzene ring or pyridine ring;

each $R^8$ is independently selected from the group consisting of hydrogen atom, deuterium atom, halogen, cyano, $C_{1-6}$ alkyl and $C_{1-6}$ alkoxy;

m is selected from the group consisting of 0, 1, 2, 3 and 4;

n is selected from the group consisting of 1, 2, 3 and 4.

In alternative embodiments, in the compound of general formula (II) or the tautomer, mesomer, racemate, enantiomer, diastereomer, atropisomer thereof, or mixture form thereof, or the pharmaceutically acceptable salt thereof, $R^1$ is selected from the group consisting of $C_{1-6}$ alkyl or halo$C_{1-6}$ alkyl, the $C_{1-6}$ alkyl or halo$C_{1-6}$ alkyl is optionally substituted by one or more deuterium atoms.

In alternative embodiments, in the compound of general formula (II) or the tautomer, mesomer, racemate, enantiomer, diastereomer, atropisomer thereof, or mixture form thereof, or the pharmaceutically acceptable salt thereof, $Y^1$ is -S-.

In alternative embodiments, in the compound of general formula (II) or the tautomer, mesomer, racemate, enantiomer, diastereomer, atropisomer thereof, or mixture form thereof, or the pharmaceutically acceptable salt thereof, wherein ring A is selected from the group consisting of phenyl and pyridyl.

In alternative embodiments, in the compound of general formula (II) or the tautomer, mesomer, racemate, enantiomer, diastereomer, atropisomer thereof, or mixture form thereof, or the pharmaceutically acceptable salt thereof, each $R^3$ is independently selected from the group consisting of hydrogen atom, deuterium atom, halogen, cyano, $C_{1-6}$ alkyl, $C_{1-6}$ alkoxy, halo$C_{1-6}$ alkyl, halo$C_{1-6}$ alkoxy, $C_{3-8}$ cycloalkyl, 3-12 membered heterocyclyl and $-NR^aR^b$; the $R^a$ and $R^b$ are each independently selected from the group consisting of hydrogen, deuterium atom, hydroxy and $C_{1-6}$ alkyl, the alkyl is substituted by one or more deuterium atoms.

In alternative embodiments, in the compound of general formula (II) or the tautomer, mesomer, racemate, enantiomer, diastereomer, atropisomer thereof, or mixture form thereof, or the pharmaceutically acceptable salt thereof, ring B is a benzene ring or pyridine ring.

In alternative embodiments, in the compound of general formula (II) or the tautomer, mesomer, racemate, enantiomer, diastereomer, atropisomer thereof, or mixture form thereof, or the pharmaceutically acceptable salt thereof,

$R^1$ is selected from methyl, the methyl is optionally substituted by one or more deuterium atoms;

$Y^1$ is -S-;

ring A is selected from the group consisting of phenyl and pyridyl;

each $R^3$ is independently selected from the group consisting of hydrogen atom, deuterium atom, halogen and $-NR^aR^b$;

the $R^a$ and $R^b$ are each independently selected from the group consisting of hydrogen, deuterium atom and $C_{1-6}$ alkyl, the alkyl is substituted by one or more deuterium atoms;

ring B is a benzene ring or pyridine ring;

m is selected from 0;

n is selected from the group consisting of 1, 2, 3 and 4.

In alternative embodiments, in the compound of general formula (II) or the tautomer, mesomer, racemate, enantiomer, diastereomer, atropisomer thereof, or mixture form thereof, or the pharmaceutically acceptable salt thereof,

$R^1$ is selected from methyl;

$Y^1$ is -S-;

ring A is selected from pyridyl;

each $R^3$ is independently selected from the group consisting of hydrogen atom, deuterium atom, halogen and $-NR^aR^b$;

the $R^a$ and $R^b$ are each independently selected from the group consisting of hydrogen, deuterium atom and $C_{1-6}$ alkyl, the alkyl is substituted by one or more deuterium atoms;

ring B is a pyridine ring;

m is selected from 0;
n is selected from the group consisting of 1, 2, 3 and 4.

In alternative embodiments, in the compound of general formula (II) or the tautomer, mesomer, racemate, enantiomer, diastereomer, atropisomer thereof, or mixture form thereof, or the pharmaceutically acceptable salt thereof,

$R^1$ is selected from methyl,
$Y^1$ is -S-;
ring A is selected from pyridyl;
each $R^3$ is independently selected from the group consisting of hydrogen atom, deuterium atom, chlorine atom, $-NH-CH_3$ and $N-(CH_3)_2$; the hydrogen atom on the methyl of the $-NH-CH_3$ or $N-(CH_3)_2$ is substituted by one or more deuterium atoms;
ring B is a pyridine ring;
m is selected from 0;
n is selected from the group consisting of 1, 2, 3 and 4.

In the present disclosure, when $Y^1$ is a direct bond, since the rotation around the bond is restrained, the compound provided by the present disclosure may exist as a mixture of atropisomers, and the enantiomeric excess is between 0-98%. When the compound is a pure atropisomer, the stereochemistry of each chiral center can be specified by aR or aS, and these names can also be used for a mixture rich in one atropisomer. The aR and aS atropisomers can be resolved by chiral chromatography.

Further description of atropisomerism and axial chirality can be found in Eliel, E.L. & Wilen, S. H. 'Stereochemistry of Organic Compounds', John Wiley and Sons, Inc. 1994.

Typical compounds of general formula (I) and general formula (II) of the present disclosure include, but are not limited to:

| Compound No. | Chemical structure and name |
|---|---|
| 1 | 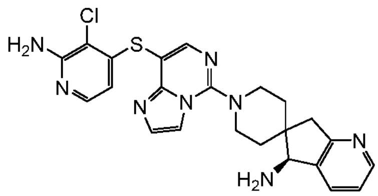 |
| | (S)-1'-(8-((2-Amino-3-chloropyridin-4-yl)thio)imidazo[1,2-c]pyrimidin-5-yl)-5,7-dihydrospiro [cyclopenta[b]pyridine-6,4'-piperidin]-5-amine |
| 2 | 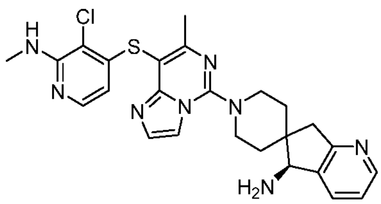 |
| | (S)-1'-(8-((3-Chloro-2-(methylamino)pyridin-4-yl)thio)-7-methylimidazo [1,2-c]pyrimidin-5-yl)-5,7-dihydrospiro[cyclopenta[b]pyridine-6,4'-piperidin]-5-amine |
| 3 | 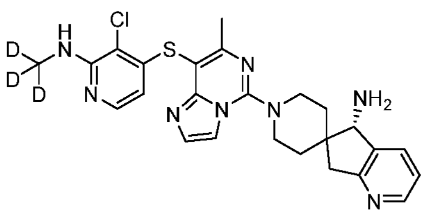 |
| | (S)-1'-(8-((3-Chloro-2-((methyl-d3)amino)pyridin-4-yl)thio)-7-methylim idazo[1,2-c]pyrimidin-5-yl)-5,7-dihydrospiro[cyclopenta[b]pyridine-6,4'-piperidin]-5-amine |

(continued)

| Compound No. | Chemical structure and name |
|---|---|
| 4 | 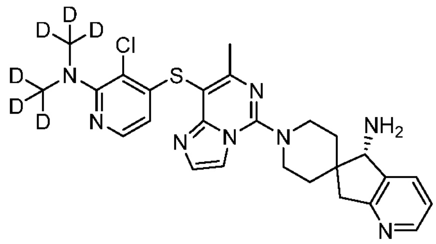 |
| | (S)-1'-(8-((2-(Bis(methyl-d3)amino)-3-chloropyridin-4-yl)thio)-7-methyl imidazo[1,2-c]pyrimidin-5-yl)-5,7-dihydrospiro[cyclopenta[b]pyridine-6,4'-piperidin]-5-amine |
| 5 | 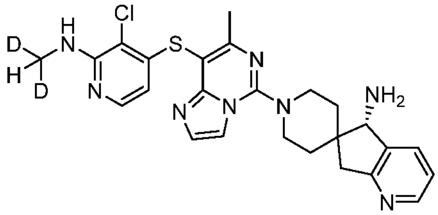 |
| | (S)-1'-(8-((3-Chloro-2-((methyl-d2)amino)pyridin-4-yl)thio)-7-methylim idazo[1,2-c]pyrimidin-5-yl)-5,7-dihydrospiro[cyclopenta[b]pyridine-6,4'-piperidin]-5-amine |
| 6 | 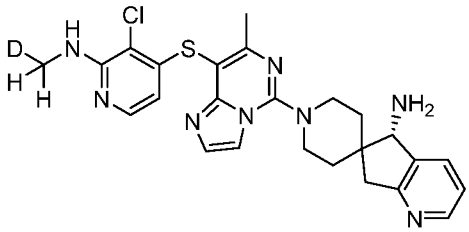 |
| | (S)-1'-(8-((3-Chloro-2-((methyl-d)amino)pyridin-4-yl)thio)-7-methylimi dazo[1,2-c]pyrimidin-5-yl)-5,7-dihydrospiro[cyclopenta[b]pyridine-6,4'-piperidin]-5-amine |
| 7 | 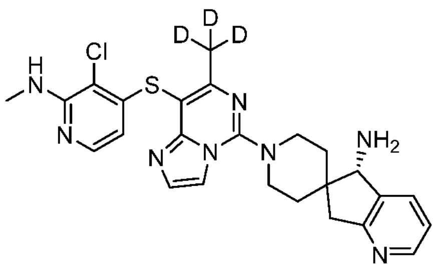 |
| | (S)-1'-(8-((3-Chloro-2-(methylamino)pyridin-4-yl)thio)-7-(methyl-d3)im idazo[1,2-c]pyrimidin-5-yl)-5,7-dihydrospiro[cyclopenta[b]pyridine-6,4'-piperidin]-5-amine |

(continued)

| Compound No. | Chemical structure and name |
|---|---|
| 8 | 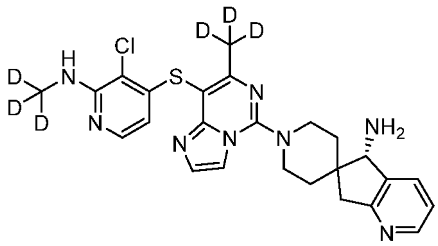<br>(S)-1'-(8-((3-Chloro-2-((methyl-d3)amino)pyridin-4-yl)thio)-7-(methyl-d 3)imidazo[1,2-c] pyrimidin-5-yl)-5,7-dihydrospiro[cyclopenta[b]pyridine-6,4'-piperidin]-5-amine |
| 9 | 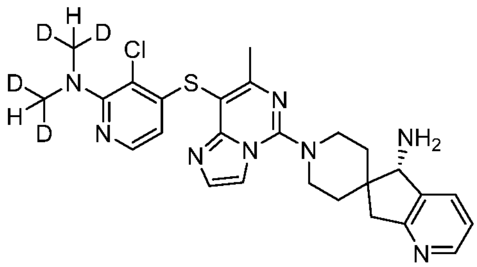<br>(S)-1'-(8-((2-(Bis(methyl-d2)amino)-3-chloropyridin-4-yl)thio)-7-methyl imidazo[1,2-c]pyrimidin-5-yl)-5,7-dihydrospiro[cyclopenta[b]pyridine-6,4'-piperidin]-5-amine |
| 10 | 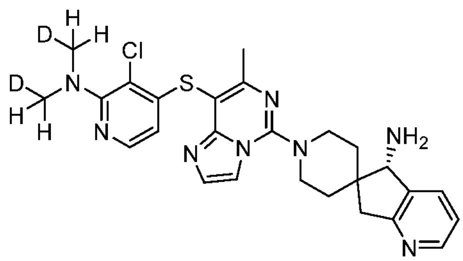<br>(S)-1'-(8-((2-(Bis(methyl-d)amino)-3-chloropyridin-4-yl)thio)-7-methyli midazo[1,2-c]pyrimidin-5-yl)-5,7-dihydrospiro[cyclopenta[b]pyridine-6,4'-piperidin]-5-amine |
| 11 | 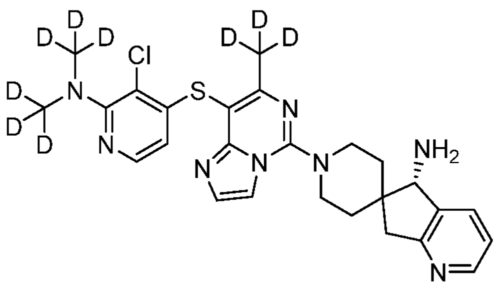<br>(S)-1'-(8-((2-(Bis(methyl-d3)amino)-3-chloropyridin-4-yl)thio)-7-(methy 1-d3)imidazo[1,2-c] pyrimidin-5-yl)-5,7-dihydrospiro[cyclopenta[b]pyridi ne-6,4'-piperidin]-5-amine |

(continued)

| Compound No. | Chemical structure and name |
|---|---|
| 12 | 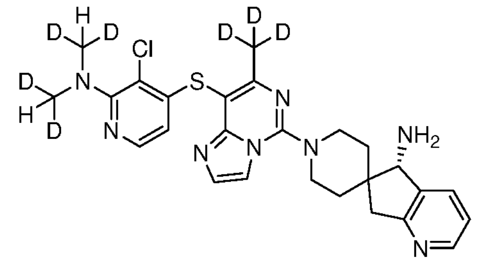 |
| | (S)-1'-(8-((2-(Bis(methyl-d2)amino)-3-chloropyridin-4-yl)thio)-7-(methy l-d3)imidazo[1,2-c] pyrimidin-5-yl)-5,7-dihydrospiro[cyclopenta[b]pyridi ne-6,4'-piperidin]-5-amine |
| 13 | 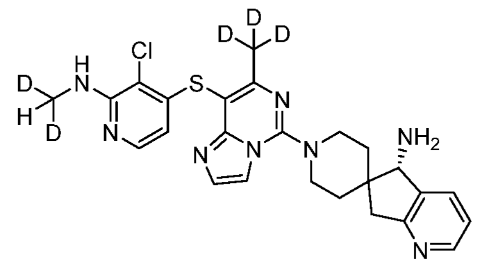 |
| | (S)-1'-(8-((3-Chloro-2-((methyl-d2)amino)pyridin-4-yl)thio)-7-(methyl-d 3)imidazo[1,2-c] pyrimidin-5-yl)-5,7-dihydrospiro[cyclopenta[b]pyridine -6,4'-piperidin]-5-amine |
| 14 | 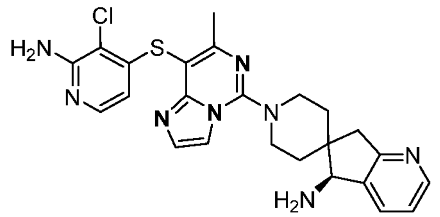 |
| | (S)-1'-(8-((2-Amino-3-chloropyridin-4-yl)thio)-7-methylimidazo[1,2-c]p yrimidin-5-yl)-5,7-dihydrospiro[cyclopenta[b]pyridine-6,4'-piperidin]-5-amine |

a tautomer, mesomer, racemate, enantiomer, diastereomer, atropisomer thereof, or mixture form thereof, or a pharmaceutically acceptable salt thereof.

The present disclosure provides a method for preparing the compound of general formula (II-1) or the tautomer, mesomer, racemate, enantiomer, diastereomer, atropisomer thereof, or mixture form thereof, or the pharmaceutically acceptable salt thereof

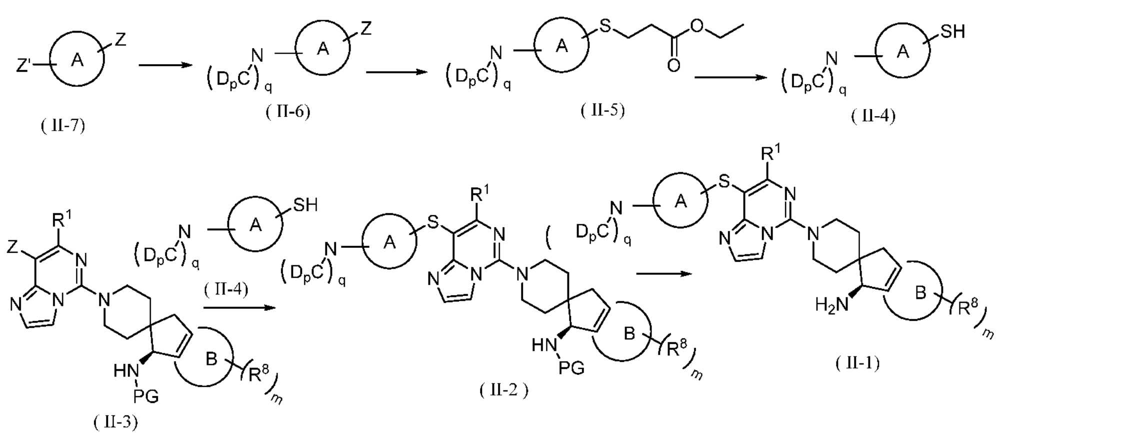

( II-7 ) ( II-6 ) ( II-5 ) ( II-4 )

( II-3 ) ( II-4 ) ( II-2 ) ( II-1 )

a compound of formula (II-7) and deuterated methylamine or deuterated dimethylamine are subjected to a substitution reaction under an alkaline condition to obtain a compound of formula (II-6);
the compound of formula (II-6) is subjected to C-S coupling under an alkaline condition to obtain a compound of formula (II-5);
the protecting group of the compound of formula (II-5) is removed under an alkaline condition to obtain a compound of formula (II-4);
the compound of formula (II-4) and a compound of formula (II-3) are subjected to C-S coupling under an alkaline condition to obtain a compound of formula (II-2);
the protecting group PG of the compound of formula (II-2) is removed to obtain the compound of formula (II-5);
wherein, the reagent that provide an alkaline condition includes organic bases and inorganic bases, the organic bases are selected from the group consisting of triethylamine, *N,N*-diisopropylethylamine, n-butyllithium, lithium diisopropylamide, lithium bistrimethylsilylamide, potassium acetate, sodium tert-butoxide and potassium tert-butoxide, the inorganic bases are selected from the group consisting of sodium hydride, potassium phosphate, sodium carbonate, potassium carbonate, sodium methoxide, sodium ethoxide, sodium tert-butoxide, potassium acetate, cesium carbonate, sodium hydroxide and lithium hydroxide;
Z and Z' are selected from the group consisting of halogen, sulfonyl and sulfinyl;
PG is selected from the group consisting of protecting groups Boc, PMB, $S(=O)^tBu$ and Cbz;
p is selected from the group consisting of 1, 2 and 3;
q is selected from the group consisting of 1 and 2;
ring A, ring B, $R^1$, $R^3$, B and m are as defined above.

The present disclosure is directed to a compound of formula (II-2) or a pharmaceutically acceptable salt thereof,

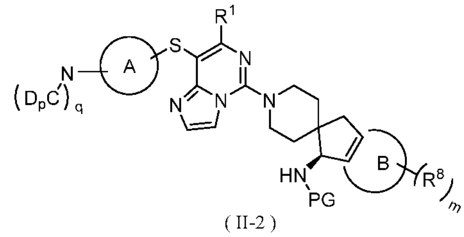

( II-2 )

wherein PG is selected from the group consisting of protecting groups Boc, PMB, $S(=O)^tBu$ and Cbz;
p is selected from the group consisting of 1, 2 and 3;
q is selected from the group consisting of 1 and 2;
ring A, ring B, $R^1$, $R^8$, B and m are as defined above.

In another aspect, the present disclosure relates to a pharmaceutical composition comprising a therapeutically effective amount of the compound of general formula (II) or the tautomer, mesomer, racemate, enantiomer, diastereomer, atropisomer thereof, or mixture form thereof, or the pharmaceutically acceptable salt, and one or more pharmaceutically acceptable carriers, diluents or excipients. The therapeutically effective amount in the present disclosure can be 0.1-2000 mg.

The present disclosure also relates to a method for preparing the pharmaceutical composition, comprising mixing the compound of general formula (I) or the tautomer, mesomer, racemate, enantiomer, diastereomer, atropisomer thereof, or mixture form thereof, or the pharmaceutically acceptable salt thereof, or the compound of general formula (II) or the tautomer, mesomer, racemate, enantiomer, diastereomer, atropisomer thereof, or mixture form thereof, or the pharmaceutically acceptable salt thereof, with a pharmaceutically acceptable carrier, diluent or excipient.

The present disclosure further relates to a use of the compound of general formula (II) or the tautomer, mesomer, racemate, enantiomer, diastereomer, atropisomer thereof, or mixture form thereof, or a pharmaceutically acceptable salt thereof, or the pharmaceutical composition comprising the same, in the preparation of a SHP2 inhibitor.

The present disclosure further relates to a use of the compound of general formula (II) or the tautomer, mesomer, racemate, enantiomer, diastereomer, atropisomer thereof, or mixture form thereof, or the pharmaceutically acceptable salt thereof, or the pharmaceutical composition comprising the same, in the preparation of a medicament for treating a disease or disorder mediated by SHP2 activity.

The present disclosure further relates to a use of the compound of general formula (II) or the tautomer, mesomer, racemate, enantiomer, diastereomer, atropisomer thereof, or mixture form thereof, or the pharmaceutically acceptable salt thereof, or the pharmaceutical composition comprising the same, as a SHP2 inhibitor in the preparation of a medicament for preventing and/or treating tumor or cancer.

The present disclosure further relates to a use of the compound of general formula (II) or the tautomer, mesomer, racemate, enantiomer, diastereomer, atropisomer thereof, or mixture form thereof, or the pharmaceutically acceptable salt thereof, or the pharmaceutical composition comprising the same, in the preparation of a medicament for preventing and/or treating Noonan syndrome, Leopard syndrome, juvenile myelomonocytic leukemia, neuroblastoma, melanoma, acute myeloid leukemia, breast cancer, esophageal cancer, lung cancer, colon cancer, head cancer, pancreatic cancer, head and neck squamous cell carcinoma, stomach cancer, liver cancer, anaplastic large cell lymphoma or glioblastoma.

The present disclosure further relates to the compound of general formula (II) or the tautomer, mesomer, racemate, enantiomer, diastereomer, atropisomer thereof, or mixture form thereof, or the pharmaceutically acceptable salt thereof, or the pharmaceutical composition comprising the same, for use as a medicament.

The present disclosure also relates to the compound of general formula (II) or the tautomer, mesomer, racemate, enantiomer, diastereomer, atropisomer thereof, or mixture form thereof, or the pharmaceutically acceptable salt thereof, or the pharmaceutical composition comprising the same, for use as a SHP2 inhibitor.

The present disclosure also relates to the compound of general formula (II) or the tautomer, mesomer, racemate, enantiomer, diastereomer, atropisomer thereof, or mixture form thereof, or the pharmaceutically acceptable salt thereof, or the pharmaceutical composition comprising the same, for use as a SHP2 inhibitor for preventing and/or treating tumor or cancer.

The present disclosure also relates to a method for preventing and/or treating tumor or cancer, comprising administering to a patient in need a therapeutically effective amount of the compound of general formula (II) or the tautomer, mesomer, racemate, enantiomer, diastereomer, atropisomer thereof, or mixture form thereof, or the pharmaceutically acceptable salt thereof, or the pharmaceutical composition comprising the same, as a SHP2 inhibitor.

The pharmaceutical composition containing an active ingredient can be in a form suitable for oral administration, for example tablet, troche, lozenge, aqueous or oily suspension, dispersible powder or granule, emulsion, hard or soft capsule, syrup or elixir. The oral composition can be prepared according to any method known in the art for preparing a pharmaceutical composition, and such a composition can contain one or more ingredients selected from the group consisting of sweeteners, flavoring agents, coloring agents and preservatives, in order to provide a pleasing and palatable pharmaceutical formulation. The tablet contains the active ingredient in admixture with non-toxic pharmaceutically acceptable excipients suitable for the manufacture of tablets. These excipients can be inert excipients, granulating agents, disintegrating agents, binders and lubricants. The tablet can be uncoated or coated by means of a known technique to mask drug taste or delay the disintegration and absorption of the active ingredient in the gastrointestinal tract, thereby providing sustained release over a longer period of time.

The oral preparation can also be provided as soft gelatin capsules, wherein the active ingredient is mixed with an inert solid diluent, or wherein the active ingredient is mixed with a water-soluble carrier or an oil solvent.

The aqueous suspension contains the active substance in admixture with excipients suitable for the manufacture of an aqueous suspension. Such excipients are suspending agents, dispersing agents or wetting agents. The aqueous suspension can also contain one or more preservatives, one or more coloring agents, one or more flavoring agents and one or more sweeteners.

The oil suspension can be formulated by suspending the active ingredient in vegetable oil or mineral oil. The oil suspension can contain a thickening agent. The above sweeteners and flavoring agents can be added to provide a palatable formulation. These compositions can be kept by adding antioxidants.

The pharmaceutical composition of the present disclosure can also be in the form of an oil-in-water emulsion. The oil phase can be vegetable oil or mineral oil or a mixture thereof. Suitable emulsifying agents can be naturally occurring phospholipids, and the emulsion can also contain a sweetener, flavoring agent, preservative and antioxidant. Such a formulation may also contain a demulcent, preservative, coloring agent, and antioxidant.

The pharmaceutical composition of the present disclosure can be in the form of a sterile injectable aqueous solution. Acceptable vehicles or solvents that can be used are water, Ringer's solution and isotonic solution of sodium chloride. The sterile injectable formulation can be a sterile injectable oil-in-water microemulsion in which the active ingredient is dissolved in the oil phase. The injectable solution or microemulsion can be injected into the bloodstream of the patient by local bolus injection. Alternatively, the solution and microemulsion are preferably administered in a manner that maintains a constant circulating concentration of the compound of the present disclosure. To maintain this constant concentration, a continuous intravenous delivery device can be used. An example of such a device is Deltec CADD-PLUS. TM. 5400 intravenous injection pump.

The pharmaceutical composition of the present disclosure can be in the form of a sterile injectable aqueous or oil suspension for intramuscular and subcutaneous administration. The suspension can be formulated with those suitable dispersing agents or wetting agents and suspending agents described above according to known techniques. The sterile injectable formulation can also be a sterile injectable solution or suspension prepared in a parenterally acceptable non-toxic diluent or solvent. Moreover, a sterile fixed oil can be conveniently used as a solvent or suspension medium. For this purpose, any blended fixed oil can be used. In addition, fatty acids can also used to prepare the injection.

The compound of the present disclosure can be administered in the form of a suppository for rectal administration. These pharmaceutical compositions can be prepared by mixing the drug with a suitable non-irritating excipient, which is solid at normal temperature but is liquid in the rectum, thereby melting in the rectum to release the drug.

As is well known to those skilled in the art, the dosage of a drug depends on a variety of factors including, but not limited to, the following factors: activity of a specific compound, age of the patient, weight of the patient, general health of the patient, behavior of the patient, diet of the patient, administration time, administration route, excretion rate, drug combination, and the like. In addition, the optimal treatment method, such as the mode of treatment, the daily dosage of the compound of the general formula (II), or the type of the pharmaceutically acceptable salt thereof, can be verified according to the traditional therapeutic regimens.

DEDINITIONS

Unless stated to the contrary, the terms used in the specification and claims have the following meanings.

The term "alkyl" refers to a saturated aliphatic hydrocarbon group, which is a straight or branched chain group comprising 1 to 20 carbon atoms, preferably an alkyl containing 1 to 12 carbon atoms, and more preferably an alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, and the like. More preferably, the alkyl group is a lower alkyl containing 1 to 6 carbon atoms, non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl can be substituted or unsubstituted. When substituted, the substituent(s) can be substituted at any available connection point. The substituent(s) is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxy and alkoxycarbonyl.

The term "cycloalkyl" refers to a saturated or partially unsaturated monocyclic or polycyclic hydrocarbon substituent group, the cycloalkyl ring comprises 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptantrienyl, cyclooctyl, and the like. Polycyclic cycloalkyl includes spiro, fused and bridged cycloalkyl.

The term "spiro cycloalkyl" refers to a 5 to 20 membered polycyclic group with individual rings connected through one shared carbon atom (called a spiro atom), wherein one or more double bonds, but none of the rings has a completely conjugated $\pi$ electron system. The spiro cycloalkyl is preferably 6 to 14 membered, and more preferably 7 to 10 membered. The spiro cycloalkyl is classified into mono-spiro cycloalkyl, di-spiro cycloalkyl and poly-spiro cycloalkyl, according to the number of the spiro atoms shared between the rings. The spiro cycloalkyl is preferably mono-spiro-cycloalkyl and di-spiro cycloalkyl, and more preferably 4-membered/4-membered, 4-membered/5-membered, 4-membered/6-membered, 5-membered/5-membered or 5-membered/6-membered mono-spiro cycloalkyl. Non-limiting examples of spiro cycloalkyl include:

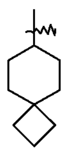 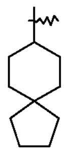 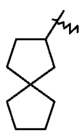 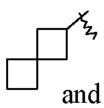 and 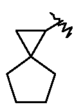 .

The term "heterocyclyl" refers to a 3 to 20 membered saturated or partially unsaturated monocyclic or polycyclic hydrocarbon substituent, wherein one or more ring atoms are heteroatoms selected from the group consisting of nitrogen, oxygen and $S(O)_m$ (wherein m is an integer from 0 to 2), but excluding -O-O-, -O-S- or -S-Sin the ring, with the remaining ring atoms being carbon atoms. It preferably comprises 3 to 12 ring atoms, 1 to 4 of which are heteroatoms; most preferably comprises 3 to 8 ring atoms, 1 to 3 of which are heteroatoms; most preferably comprises 3 to 6 ring atoms,

1 to 2 of which are heteroatoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, preferably piperidinyl, piperazinyl or morpholinyl. Polycyclic heterocyclyl includes spiro, fused and bridged heterocyclyl.

The heterocyclic ring can be fused to the ring of aryl, heteroaryl or cycloalkyl, wherein the ring attached to the parent structure is the heterocyclyl, non-limiting examples thereof include:

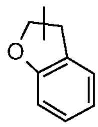 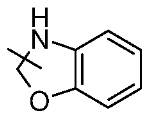 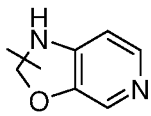 and 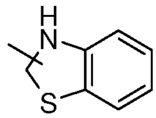

and the like.

The term "aryl" refers to a 6 to 14 membered all-carbon monocyclic or fused polycyclic (i.e. each ring in the system shares an adjacent pair of carbon atoms with another ring in the system) group having a conjugated $\pi$ electron system, preferably 6 to 10 membered, for example phenyl and naphthyl, and more preferably phenyl. The aryl ring can be fused to the ring of heteroaryl, heterocyclyl or cycloalkyl, wherein the ring attached to he parent structure is the aryl ring, non-limiting examples thereof include:

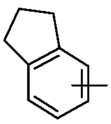 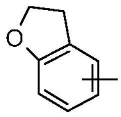 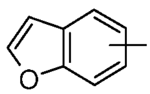 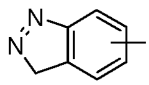 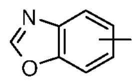 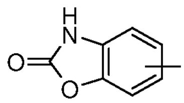 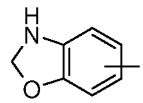

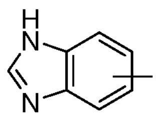 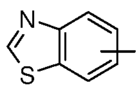 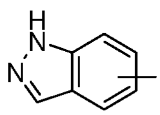 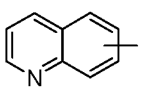 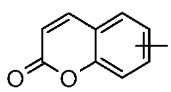 and 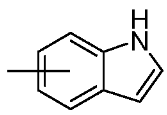 .

The aryl can be substituted or unsubstituted. When substituted, the substituent(s) is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxy and alkoxycarbonyl.

The term "heteroaryl" refers to a 5 to 14 membered heteroaromatic system comprising 1 to 4 heteroatoms selected from the group consisting of oxygen, sulfur and nitrogen. The heteroaryl is preferably 5 to 10 membered, comprising 1 to 3 heteroatoms; more preferably 5 or 6 membered, comprising 1 to 2 heteroatoms; preferably, for example, imidazolyl, furyl, thienyl, thiazolyl, pyrazolyl, oxazolyl, pyrrolyl, tetrazolyl, pyridyl, pyrimidinyl, thiadiazole, pyrazinyl and the like; preferably imidazolyl, tetrazolyl, pyridyl, thienyl, pyrazolyl, pyrimidinyl, or thiazolyl; more preferably pyridyl. The heteroaryl ring can be fused to the ring of aryl, heterocyclyl or cycloalkyl, wherein the ring attached to the parent structure is the heteroaryl ring. Non-limiting examples thereof include:

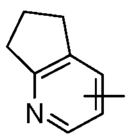 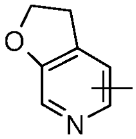 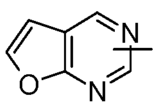 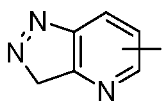 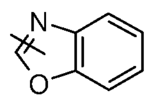 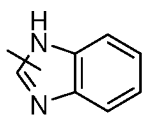

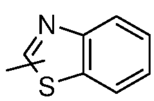 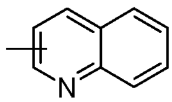 and 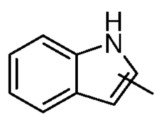 .

The heteroaryl can be optionally substituted or unsubstituted. When substituted, the substituent(s) is preferably one or more groups independently selected from the group consisting of alkyl, alkenyl, alkynyl, alkoxy, alkylthiol, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxy and alkoxycarbonyl.

The term "halogen" refers to fluorine, chlorine, bromine or iodine.

The term "haloalkyl" refers to an alkyl substituted by one or more halogens, wherein the alkyl is as defined above.

The term "haloalkoxy" refers to an alkoxy substituted by one or more halogens, wherein the alkoxy is as defined above.

The term "hydroxyalkyl" refers to an alkyl substituted by hydroxy(s), wherein the alkyl is as defined above.

The term "alkylamino" refers to an amino substituted by one or two alkyl(s), wherein the alkyl is as defined above.

The term "hydroxy" refers to an -OH group.

The term "halogen" refers to fluorine, chlorine, bromine or iodine.

The term "amino" refers to a $-NH_2$ group.

The term "cyano" refers to a -CN group.

The term "nitro" refers to a $-NO_2$ group.

The term "oxo" refers to a =O group.

The term "carbonyl" refers to a C=O group.

The term "carboxy" refers to a -C(O)OH group.

The term "thio" refers to -S-.

The term "sulfhydryl" refers to -SH.

"Optional" or "optionally" means that the subsequently described event or circumstance can, but need not occur, and such a description includes the situation where the event or circumstance occurs or does not occur. For example, "the heterocyclyl optionally substituted by an alkyl" means that the alkyl group can be, but need not be, present, and such a description includes the situation of the heterocyclyl being substituted by an alkyl and the heterocyclyl being not substituted by an alkyl.

"Substituted" refers to one or more hydrogen atoms, preferably at most 5, more preferably 1 to 3 hydrogen atoms in a group, independently substituted by a corresponding number of substituents. It goes without saying that the substituents are only in their possible chemical positions. Those skilled in the art are able to determine (by experiment or theory) whether the substitution is possible or impossible without excessive effort. For example, the binding of an amino or a hydroxy with free hydrogen to a carbon atom with unsaturated (such as olefinic) bond may be unstable.

"Pharmaceutical composition" represents a mixture containing one or more of the compounds described herein, or a physiologically/pharmaceutically acceptable salt or prodrug thereof, and other chemical components, as well as other components, for example, physiological/pharmaceutically acceptable carriers and excipients. The purpose of the pharmaceutical composition is to promote drug administration to organisms, to facilitate the absorption of the active ingredient and thereby exerting the biological activity.

"Pharmaceutically acceptable salt" refers to a salt of the compound of the present disclosure, which is safe and effective for use in mammals *in vivo* and has the desired biological activity.

"One or more" refers to the number optionally selected from the group consisting of 1, 2, 3, 4, 5 and 6.

## DETAILED DESCRIPTION

The present disclosue will be further described with reference to the following examples, but the examples should not be considered as limiting the scope of the present disclosure.

## EXAMPLES

The structure of the compounds were determined by nuclear magnetic resonance (NMR) or/and mass spectrometry (MS). The NMR shift ($\delta$) was given in the unit of $10^{-6}$ (ppm). Bruker AVANCE-400 nuclear magnetic spectrometer was used for NMR determination. The solvents for determination were deuterated dimethyl sulfoxide (DMSO-$d_6$), deuterated chloroform ($CDCl_3$), deuterated methanol ($CD_3OD$), and the internal standard was tetramethylsilane (TMS).

Shimadzu 2010 Mass Spectrometer or Agilent 6110A MSD mass spectrometer was used for MS determination.

Shimadzu LC-20A systems, Shimadzu LC-2010HT series or Agilent 1200 LC high pressure liquid chromatograph (Ultimate XB-C18 3.0*150mm column or Xtimate C18 2.1*30mm column) were used for high performance liquid chromatography (HPLC) determination.

Chiralpak IC-3 100×4.6 mm I.D., 3 μm, Chiralpak AD-3 150×4.6 mm I.D., 3 μm, Chiralpak AD-3 50×4.6 mm I.D., 3 μm, Chiralpak AS-3 150×4.6 mm I.D., 3 μm, Chiralpak AS-3 100×4.6 mm I.D., 3 μm, ChiralCel OD-3 150×4.6 mm I.D., 3 μm, Chiralcel OD-3 100×4.6 mm I.D., 3 μm, ChiralCel OJ-H 150×4.6 mm I.D., 5 μm, Chiralcel OJ-3 150×4.6 mm I.D., 3 μm columns were used for determination of Chiral HPLC analysis;

As for the thin layer chromatography silica gel plates, Yantai Huanghai HSGF254 or Qingdao GF254 silica gel plates were used. The dimension of the silica gel plates used in TLC was 0.15 mm to 0.2 mm, and the dimension of the

silica gel plates used in product purification by thin-layer chromatography was 0.4 mm to 0.5 mm.

Yantai Huanghai silica gel of 100 to 200 mesh, 200 to 300 mesh or 300 to 400 mesh were generally used as carrier in column chromatography.

As for chiral preparative columns, DAICEL CHIRALPAK IC (250 mm*30 mm, 10 μm) or Phenomenex-Amylose-1 (250 mm*30 mm, 5 μm) was used.

As for CombiFlash rapid preparation instrument, Combiflash Rf150 (TELEDYNE ISCO) was used.

The average kinase inhibition rate and $IC_{50}$ value were determined by using NovoStar microplate reader (BMG, German).

The known starting materials of the present disclosure can be synthesized by or according to methods known in the art, or can be purchased from ABCR GmbH & Co. KG, Acros Organics, Aldrich Chemical Company, Accela ChemBio Inc, Darui Chemicals and other companies.

Unless otherwise specified in the examples, the reactions are all able to be carried out under argon atmosphere or nitrogen atmosphere.

Argon atmosphere or nitrogen atmosphere means that the reaction flask is equipped with an argon or nitrogen balloon with a volume of about 1 L.

Hydrogen atmosphere means that the reaction flask is equipped with a hydrogen balloon with a volume of about 1 L.

Parr 3916EKX hydrogenator and Qinglan QL-500 hydrogen generator or HC2-SS hydrogenator were used for pressurized hydrogenation reaction.

As for hydrogenation reaction, the reaction system was generally vacuumized and filled with hydrogen, and the above operation was repeated for 3 times.

CEM Discover-S 908860 microwave reactor was used for microwave reaction.

Unless otherwise specified in the examples, the solution refers to an aqueous solution.

Unless otherwise specified in the examples, the reaction temperature is room temperature from 20°C to 30°C.

The reaction process in the examples was monitored by thin layer chromatography (TLC). The developing reagent used in the reactions, the eluent system in column chromatography and the developing solvent system in thin layer chromatography for purification of the compounds included: A: dichloromethane/methanol system, B: n-hexane/ethyl acetate system, C: petroleum ether/ethyl acetate system, and D: petroleum ether/ethyl acetate/methanol. The volume ratio of solvents were adjusted according to different polarity of the compounds, and a small amount of triethylamine, acetic acid or other alkaline or acidic reagents could also be added for adjustment.

Example 1

(S)-1'-(8-((2-Amino-3-chloropyridin-4-yl)thio)imidazo[1,2-c]pyrimidin-5-yl)-5,7-dih ydrospiro[cyclopenta[b]pyridine-6,4'-piperidin]-5-amine

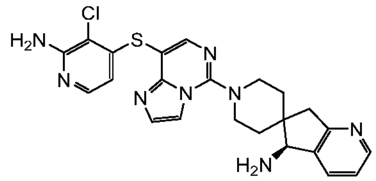

1

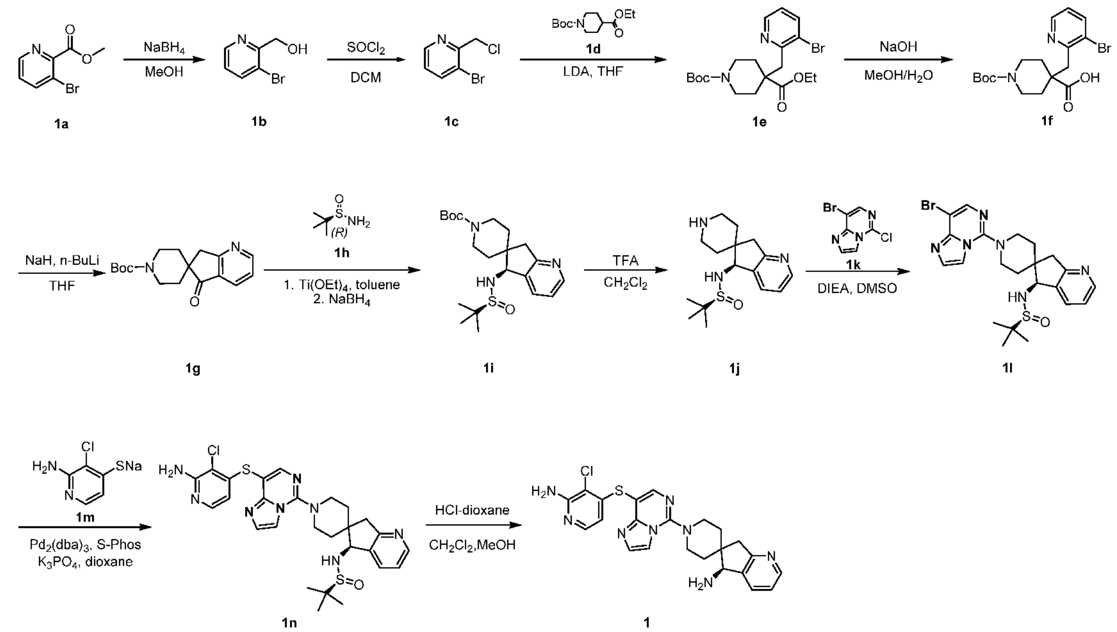


1a 1b 1c 1e 1f

1g 1i 1j 1l

1n 1

**Step 1**

(3-Bromopyridin-2-yl)methanol **1b**

Compound **1a** (17.2 g, 79.6 mmol) was dissolved in methanol (50 mL), and sodium borohydride (15.1 g, 398 mmol) was added thereto at 0°C. The reaction system was stirred at room temperature for 12 hours. After completion of the reaction, saturated aqueous solution of ammonium chloride (600 mL) was added, and the reaction solution was extracted with ethyl acetate (200 mL×3). The organic phases were combined, washed with saturated sodium chloride (200 mL × 2), dried over anhydrous sodium sulfate, and concentrated under reduced pressure to obtain compound **1b** (9.7 g, yield: 64.8%) as a white solid.

MS(ESI) m/z 187.8 $[M+H]^+$

$^1H$ NMR: (400MHz, MeOD-$d_4$) δ = 8.52 (d, J = 4.8 Hz, 1H), 8.01 (dd, J = 1.2, 8.0 Hz, 1H), 7.26 (dd, J = 4.4, 6.4 Hz, 1H), 4.77 (s, 2H).

Step 2

3-Bromo-2-(chloromethyl)pyridine **1c**

Compound **1b** (9.70 g, 51.6 mmol) was dissolved in dichloromethane (20 mL), and thionyl chloride (7.48 mL, 103 mmol) was added thereto at room temperature, followed by stirring at room temperature for 3 hours. After completion of the reaction, saturated aqueous sodium bicarbonate solution (300 mL) was added at 0°C, and the reaction solution was extracted with dichloromethane (80 mL×3). The organic phases were combined, washed with saturated sodium chloride (100 mL), dried with anhydrous sodium sulfate, and concentrated under reduced pressure to obtain compound **1c** (10.3 g, yield: 96.9%) as a pink oil.

MS(ESI) m/z 207.7 $[M+H]^+$

$^1H$ NMR (400MHz, Methanol-*d*4) δ = 8.55-8.45 (m, 1H), 8.12-7.99 (m, 1H), 7.37-7.21 (m, 1H), 4.84-4.80 (m, 2H).

Step 3

1-(Tert-butyl) 4-ethyl 4-((3-bromopyridin-2-yl)methyl)piperidine-1,4-dicarboxylate **1e**

Compound **1c** (9.97 g, 38.7 mmol) was dissolved in tetrahydrofuran (80 mL), and LDA (13.5 mL, 2 M in tetrahydrofuran and n-hexane) was added thereto at -78°C under nitrogen atmosphere. After completion of the addition, the reaction solution was stirred at -78°C for 1 hour. Then compound **1d** (8.8 g, 35.07 mmol) was added dropwise at -78°C, and the reaction solution was stirred at -78°C for 9 hours. After completion of the reaction, saturated aqueous ammonium chloride solution (400 mL) was added, and the reaction solution was extracted with ethyl acetate (100 mL × 3). The organic phases were combined, washed with saturated sodium chloride solution (100 mL × 2), dried over anhydrous sodium sulfate, and concentrated under vacuum to obtain the crude product, which was purified by silica gel chromatography with petroleum ether and ethyl acetate as eluents to obtain compound **1e** (14.8 g, yield: 89.4%) as a yellow oil.

MS(ESI) m/z 429.0 $[M+H]^+$

Step 4

4-((3-Bromopyridin-2-yl)methyl)-1-(tert-butoxycarbonyl)piperidine-4-carboxylic acid **1f**

Compound **1e** (14.8 g, 34.6 mmol) was dissolved in methanol (3 mL), and aqueous sodium hydroxide solution (13.8 g, 346 mmol, dissolved in 40 mL water) was added thereto at 0°C, followed by stirring at 80°C for 12 hours. After completion of the reaction, the reaction solution was concentrated, and ethyl acetate (300 mL) and water (300 mL) were added thereto. Saturated aqueous sodium hydroxide solution (10 mL) was added to adjust the pH to 12. The aqueous phase was separated and washed with ethyl acetate (80 mL×2). 2 N hydrochloric acid (25 mL) was added to the obtained aqueous phase to adjust the pH to 3, and the reaction solution was extracted with ethyl acetate (100 mL×3). The organic phases were combined, washed with saturated sodium chloride solution (150 mL), dried over anhydrous sodium sulfate, and concentrated under reduced pressure to obtain compound **1f** (11.4 g, yield: 82.4%) as a white solid.

MS(ESI) m/z 344.0 $[M-56+H]^+$

Step 5

Tert-butyl 5-carbonyl-5,7-dihydrospiro[cyclopenta[b]pyridine-6,4'-piperidine]-1'-carboxylate **1g**

Sodium hydride (60% kerosene mixture, 1.32 g, 33.1 mmol) was added to compound **1f** (11.0 g, 27.6 mmol) in tetrahydrofuran (100 mL) at -15°C under nitrogen atmosphere. The reaction solution was stirred at -15°C for 1 hour. Then the reaction solution was cooled to -78°C, and 2.5 M n-butyllithium solution in n-hexane (16.5 mL, 41.3 mmol) was added thereto, followed by stirring at -78°C for 1 hour. After completion of the reaction, saturated aqueous ammonium chloride solution (400 mL) was added at 0°C, and the reaction solution was extracted with ethyl acetate (100 mL×3). The organic phases were combined, washed with saturated sodium chloride (100 mL×2), dried with anhydrous sodium sulfate, and concentrated under vacuum to obtain the crude product, which was purified by silica gel chromatography with dichloromethane and methanol as eluents to obtain compound **1g** (4.60 g, yield: 55.2%) as a white solid.

MS(ESI) m/z 246.9 $[M-56+H]^+$.

$^1H$ NMR (400MHz, Methanol-*d*4) $\delta$ = 8.82 (dd, J = 1.6, 4.8 Hz, 1H), 8.12 (dd, J = 1.6, 7.6 Hz, 1H), 7.50 (dd, J = 4.8, 7.6 Hz, 1H), 4.08 (td, J = 3.6, 13.6 Hz, 2H), 3.25 (s, 2H), 3.12 (br s, 2H), 1.88-1.77 (m, 2H), 1.51 (br s, 2H), 1.49 (s, 9H).

Step 6

Tert-butyl (S)-5-((S)-tert-butylsulfinamido)-5,7-dihydrospiro[cyclopenta[b]pyridine-6,4'-piperidi ne]-1'-carboxylate **1i**

Tetraethyl titanate (9.4 mL, 44.6 mmol) was added to compound **1g** (4.50 g, 14.9 mmol) in anhydrous toluene (80 mL) under nitrogen atmosphere, and the reaction solution was stirred at room temperature for 10 minutes. Then compound **1h** (5.4 g, 44.6 mmol) was added, and the reaction solution was reacted at 120°C for 5 hours. The reaction solution was cooled to 0°C, lithium borohydride (1.58 g, 89.2 mmol) was added thereto, followed by stirring for 30 minutes. Then the reaction solution was warmed up to room temperature and stirred for 1 hour. After completion of the reaction, methanol (20 mL) was added dropwise thereto at 0°C, and then water (100 mL) and ethyl acetate (100 mL) were added, followed by stirring for 5 minutes. The reaction solution was filtered by diatomaceous earth to remove the suspended matter and washed with ethyl acetate (300 mL) and water (300 mL). The organic phases were combined, washed with saturated sodium chloride (500 mL), dried with anhydrous sodium sulfate, and concentrated under reduced pressure to

obtain the crude product, which was purified by silica gel chromatography with petroleum ether and ethyl acetate as eluents to obtain compound **1i** (4.40 g, yield: 72.6%) as a yellow solid.

MS(ESI) m/z 408.1 [M+H] $^+$

Step 7

(S)-N-((S)-5,7-Dihydrospiro[cyclopenta[b]pyridine-6,4'-piperidin]-5-yl)-2-methylpro pane-2-sulfenamide **1j**

Compound **1i** (4.40 g, 10.8 mmol) was dissolved in dichloromethane (15 mL), and trifluoroacetic acid (5 mL) was added thereto at 0°C, followed by stirring at 0°C for 1 hour. The reaction solution was concentrated under reduced pressure to obtain the crude product. 4 M aqueous sodium hydroxide solution was added thereto to pH=11, followed by extracting with chloroform and isopropanol (volume ratio 3:1) (30 mL × 3). The organic phases were combined, dried over anhydrous sodium sulfate, and concentrationed under reduced pressure to obtain the product **1j** (3.32 g, yield: 100%) as a yellow oil.

MS(ESI) m/z 307.9 [M+H]$^+$

Step 8

(S)-N-((S)-1'-(8-Bromoimidazo[1,2-c]pyrimidin-5-yl)-5,7-dihydrospiro[cyclopenta[b] pyridine-6,4'-piperidin)-5-yl)-2-methylpropane-2-sulfenamide **1l**

Compound **1j** (3.30 mg, 10.7 mmol) and compound **1k** (2.50 g, 10.7 mmol) were dissolved in dimethyl sulfoxide (40 mL) under nitrogen atmosphere, and diisopropylethylamine (7.7 g, 59.8 mmol) was added thereto, followed by stirring at 90°C for 2 hours. Ethyl acetate (50 mL) and water (100 mL) were added, and the reaction solution was extracted with ethyl acetate (50 mL × 2). The organic phases were combined, washed with saturated sodium chloride solution (50 mL × 3), dried over anhydrous sodium sulfate, and concentrated under reduced pressure to obtain the crude product, which was purified by silica gel chromatography with dichloromethane and methanol as eluents to obtain compound **1l** (2.96 g, yield: 54.6%).

MS(ESI) m/z 503.1 [M+H] $^+$

$^1$H NMR (400MHz, METHANOL-*d*4) δ = 8.41 (d, *J*=4.8 Hz, 1H), 7.97 (s, 1H), 7.92 (d, *J*=1.5 Hz, 1H), 7.81 (d, *J*=7.5 Hz, 1H), 7.66 (d, *J*=1.5 Hz, 1H), 7.32 (dd, *J*=5.0, 7.5 Hz, 1H), 4.61 (br s, 2H), 3.95 - 3.83 (m, 2H), 3.30 - 3.21 (m, 2H), 2.99 (d, *J*=16.6 Hz, 1H), 2.40 (dt, *J*=4.0, 12.7 Hz, 1H), 2.14 (dt, *J*=3.6, 12.4 Hz, 1H), 1.82 (br d, *J*=13.3 Hz, 1H), 1.54 (br d, *J*=12.3 Hz, 1H), 1.36 (s, 9H).

Step 9

(S)-N-((S)-1'-(8-((2-Amino-3-chloropyridin-4-yl)thio)imidazo[1,2-c]pyrimidin-5-yl)-5,7-dihydrospiro[cyclopenta[b]pyridine-6,4'-piperidin]-5-yl)-2-methylpropane-2-sulfe namide **1n**

Compound **1l** (70 mg, 0.14 mmol) and compound **1m** (33 mg, 0.21 mmol, prepared by the method disclosed in the patent application "WO2015107495 A1") were dissolved in 1,4-dioxane (1 mL) under nitrogen atmosphere, and diisopropylethylamine (54 mg, 0.42 mmol) was added thereto at room temperature. Tris(dibenzylideneacetone)dipalladium (13 mg, 0.014 mmol) and 2-dicyclohexylphosphino-2',6'-dimethoxybiphenyl (14 mg, 0.028 mmol) were added, and the reaction solution stirred under heating at 110°C for 12 hours. After completion of the reaction, the reaction solution was filtered, and the obtained filtrate was concentrated. The residues were purified by C-18 reverse phase chromatography with water and methanol as eluents to obtain compound **1n** (45 mg, yield: 55.1%) as a brown oil.

MS(ESI) m/z 583.1 [M+H]$^+$

Step 10

(S)-1'-(8-((2-Amino-3-chloropyridin-4-yl)thio)imidazo[1,2-c]pyrimidin-5-yl)-5,7-dih ydrospiro[cyclopenta[b]pyridine-6,4'-piperidin]-5-amine **1**

Compound **1n** (25 mg, 0.035 mmol) was dissolved in 1,4-dioxane, and a solution of hydrogen chloride in 1,4-dioxane (0.2 mL, 4 N) was added thereto at 0°C, followed by reacting at 2-7°C for 1 hour. After completion of the reaction, water (30 mL) was added, and the reaction solution was extracted with ethyl acetate (15 mL×2). The organic phases were combined, washed with saturated sodium chloride solution (20 mL), dried over anhydrous sodium sulfate and concentrated under reduced pressure. The residue was purified by C-18 reverse phase chromatography to obtain

compound **1** (3.9 mg, yield: 19.0%).

MS(ESI) m/z 479.1 $[M+H]^+$

$^1H$ NMR: (400 MHz, MeOD-$d_4$) $\delta$ = 8.38 (d, *J*= 4.8 Hz, 1H), 8.06 (s, 1H), 7.90-7.84 (m, 2H), 7.57 (s, 1H), 7.50 (d, *J* = 5.2 Hz, 1H), 7.30 (dd, *J* = 5.6, 7.6 Hz, 1H), 5.90 (d, *J* = 6.0 Hz, 1H), 4.16 (s, 1H), 4.06 (br d, *J* = 13.6 Hz, 2H), 3.48-3.36 (m, 2H), 3.30-3.24(m, 1H), 3.01(br d, *J* = 16.4 Hz, 1H), 2.20-2.01 (m, 2H), 1.80-1.71 (m, 1H), 1.61-1.53 (m, 1H).

Example 2

(S)-1'-(8-((3 -Chloro-2-(methylamino)pyridin-4-yl)thio)-7-methylimidazo[1,2-c]pyrim idin-5-yl)-5,7-dihydrospiro[cyclopenta[b]pyridine-6,4'-piperidin]-5-amine

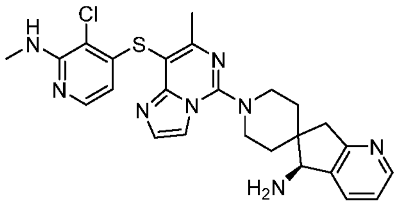

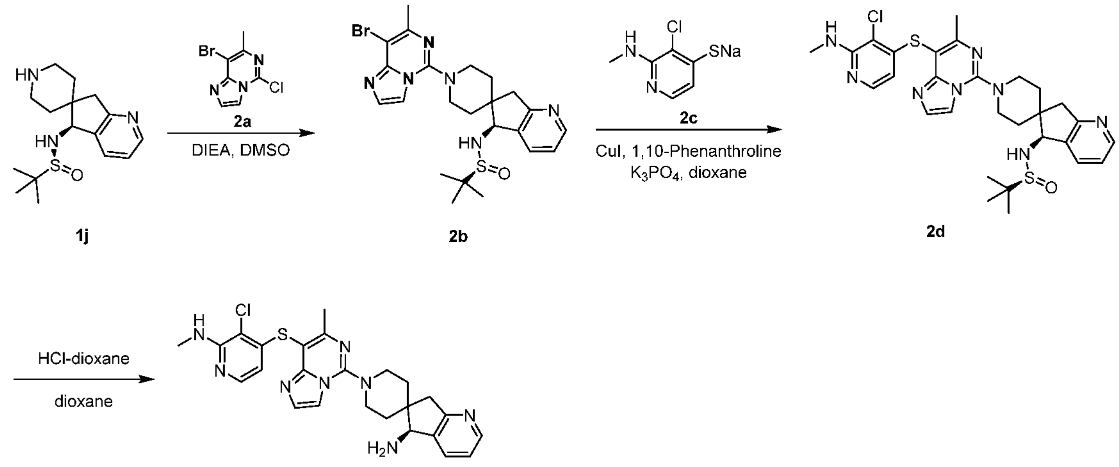

1j 2b 2d

2

Step 1

(S)-N-((S)-1'-(8-Bromo-7-methylimidazo[1,2-c]pyrimidin-5-yl)-5,7-dihydrospiro[cycl openta[b]pyridine-6,4'-piperidin]-5-yl)-2-methylpropane-2-sulfenamide **2b**

Compound **1j** (260 mg, 0.85 mmol) and compound **2a** (271 mg, 1.10 mmol) were dissolved in dimethyl sulfoxide (3 mL), and diisopropylethylamine (547 mg, 4.23 mmol) was added thereto, followed by stirring at 90°C for 1 hour. Water (30 mL) was added, and the reaction solution was extracted with ethyl acetate (30 mL×3). The organic phases were combined, washed with saturated sodium chloride solution (50 mL×2), dried over anhydrous sodium sulfate and filtered. The filtrate was concentrated under reduced pressure to obtain the crude product, which was purified by silica gel chromatography with methanol and dichloromethane as eluents to obtain compound **2b** (370 mg, yield: 84.5%).

MS(ESI) m/z 518.8 $[M+H]^+$

1H NMR (400MHz, Methanol-*d*4) $\delta$ = 8.39 (d, *J* = 4.8 Hz, 1H), 7.82 (d, *J* = 1.2 Hz, 1H), 7.79 (d, *J* = 8.0 Hz, 1H), 7.55 (d, *J* = 1.6 Hz, 1H), 7.30 (dd, *J* = 4.8 Hz, 7.6 Hz, 1H), 3.90-3.81 (m, 2H), 3.37-3.32 (m, 1H), 3.29-3.17 (m, 3H), 3.00-2.92 (m, 1H), 2.57 (s, 3H), 2.37 (td, *J* = 4.4 Hz, 12.8 Hz, 1H), 2.13 (td, *J* = 4.4 Hz, 13.2 Hz, 1H), 1.83-1.75 (m, 1H), 1.56-1.49 (m, 1H), 1.34 (s, 9H).

Step 2

(S)-N-((S)-1'-(8-((3 -chloro-2-(methylamino)pyridin-4-yl)thio)-7-methylimidazo[1,2-c ]pyrimidin-5-yl)-5,7-dihydrospiro[cyclopenta[b]pyridine-6,4'-piperidin]-5-yl)-2-meth ylpropane-2-sulfenamide **2d**

Compound **2b** (50 mg, 0.10 mmol), compound **2c** (77 mg, 0.39 mmol) and potassium phosphate (41 mg, 0.19 mmol) were dissolved in 1,4-dioxane (1 mL). Nitrogen purge was performed three times under stirring. 1,10-Phenanthroline (3.5 mg, 0.02 mmol) and cuprous iodide (1.8 mg, 0.01 mmol) were rapidly added thereto under nitrogen atmosphere. Then nitrogen purge was performed three times, and the reaction solution was stirred under heating at 130°C for 10 hours. Water (50 mL) was added, and the reaction solution was extracted with ethyl acetate (40 mL×3). The organic phases were combined, washed with saturated sodium chloride solution (70 mL×2), dried over anhydrous sodium sulfate and filtered. The filtrate was concentrated under reduced pressure to obtain the crude product, which was purified by silica gel chromatography with dichloromethane and methanol as eluents to obtain compound **2d** (36 mg, yield: 58.5%).

MS(ESI) m/z 611.1 $[M+H]^+$

Step 3

(S)-1'-(8-((3-chloro-2-(methylanino)pyridin-4-yl)thio)-7-methylimidazo[1,2-c]pyrimi din-5-yl)-5,7-dihydrospiro[cyclopenta[b]pyridine-6,4'-piperidin]-5-amine **2**

Compound **2d** (36 mg, 0.059 mmol) was dissolved in dry dioxane (1 mL), and a solution of hydrogen chloride in 1,4-dioxane (1 mL, 4 N) was added dropwise at 10°C, followed by reacting at 10°C for 15 minutes. Water (30 mL) was added, and the reaction suspension was extracted with ethyl acetate (30×3). The aqueous phase was adjusted to pH = 8 with saturated aqueous sodium bicarbonate solution, and then extracted with chloroform (40 mL×4). All organic phases were combined, dried over anhydrous sodium sulfate and filtered. The filtrate was concentrated under reduced pressure to obtain the crude product, which was purified by high performance liquid chromatography and lyophilized to obtain compound **2** (2.3 mg, yield: 7.7%).

MS(ESI) m/z 507.3 $[M+H]^+$

1H NMR (400MHz, Methanol-*d*4) δ = 8.35 (d, *J* = 4.4 Hz, 1H), 7.85 (d, *J* = 7.6 Hz, 1H), 7.76 (d, *J* = 1.6 Hz, 1H), 7.58 (d, *J* = 5.6 Hz, 1H), 7.48 (d, *J* = 1.6 Hz, 1H), 7.29 (dd, *J* = 5.2 Hz, 7.6 Hz, 1H), 5.75 (d, *J* = 6.0 Hz, 1H), 4.12-4.00 (m, 3H), 3.46-3.34 (m, 2H), 3.29-3.23 (m, 1H), 3.01-2.92 (m, 4H), 2.55 (s, 3H), 2.17-2.01 (m, 2H), 1.74 (d, *J* = 13.6 Hz, 1H), 1.53 (d, *J* = 13.6 Hz, 1H).

Example 3

(S)-1'-(8-((3-Chloro-2-((methyl-d3)amino)pyridin-4-yl)thio)-7-methylimidazo[1,2-c]p yrimidin-5-yl)-5,7-dihydrospiro[cyclopenta[b]pyridine-6,4'-piperidin]-5-amine

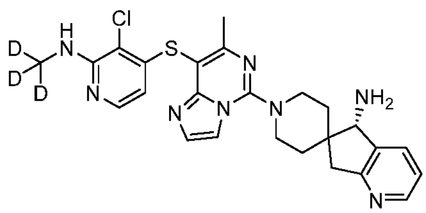

Intermediate 3e

Sodium 2-((methyl-$d_3$)amino)-3-chloropyridine-4-thiolate

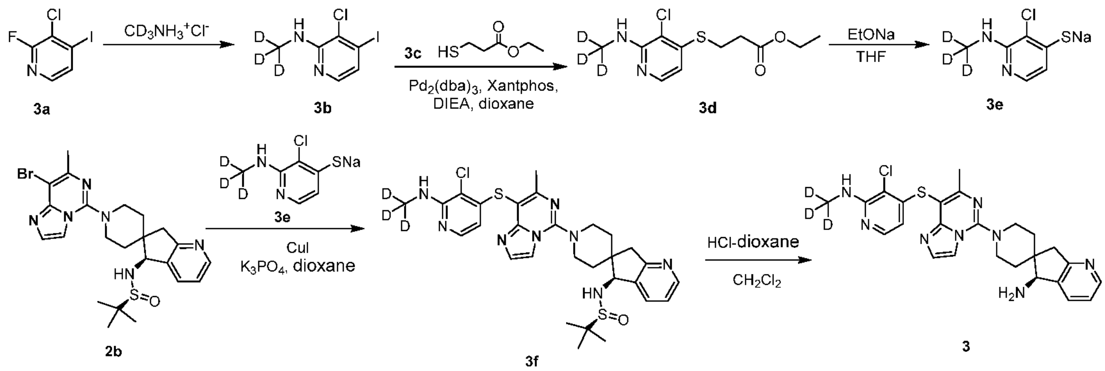


Step 1

3-Chloro-4-iodo-N-(methyl-*d*$_3$)pyridin-2-amine 3b

Compound 3a (3.0 g, 12 mmol) and methyl-*d*$_3$-amine hydrochloride (1.2 g, 16 mmol) were dissolved in DMSO (50 mL), and DIEA (5.8 mL, 35 mmol) was added thereto. The reaction solution was reacted at 70°C for 12 hours. After completion of the reaction, an ice-water mixture (50 mL) was added, and the reaction solution was filtered and washed with ice water (50 mL×3). The obtained solid was dried under reduced pressure to obtain compound **3b** (2.8 g, yield: 83%).

MS(ESI) m/z 272.0 [M+H] $^+$

$^1$H NMR: (400MHz, $CDCl_3$) δ = 7.67 (d, J = 5.2 Hz, 1H), 7.02 (d, J = 5.2 Hz, 1H), 5.14 (br s, 1H).

Step 2

Ethyl 3-((3-chloro-2-((methyl-*d*$_3$)amino)pyridin-4-yl)thio)propionate **3d** Compound **3b** (2.7 g, 10 mmol) was dissolved in dichloromethane (30 mL), and ethyl 3-thiopropionate **3c** (2.0 g, 15 mmol), tris(dibenzylideneacetone) (0.46 g, 0.50 mmol), 4,5-bis(diphenylphosphino)-9,9-dimethylxanthene (0.58 g, 0.99 mmol) and N,N-diisopropylethylamine (4.9 mL, 30 mmol) were added thereto. The reaction solution was reacted at 100°C under nitrogen atmosphere for 3 hours. After completion of the reaction, the reaction solution was filtered. The filtrate was concentrated under reduced pressure. The obtained residues were purified by silica gel chromatography with ethyl acetate and petroleum ether as eluents to obtain compound **3d** (2.5 g, yield: 90%).

MS(ESI) m/z 278.1 [M+H] $^+$

$^1$H NMR (400MHz, $CDCl_3$) δ = 7.96 (d, J = 5.6 Hz, 1H), 6.45 (d, J = 5.6 Hz, 1H), 5.00 (br s, 1H), 4.19 (q, J = 7.2 Hz, 2H), 3.23 (t, J = 7.6 Hz, 2H), 2.72 (t, J = 7.6 Hz, 2H), 1.28 (t, J = 7.2 Hz, 3H).

Step 3

Sodium 2-((methyl-*d*$_3$)amino)-3-chloropyridine-4-thiolate Intermediate **3e** Compound **3d** (2.4 g, 8.6 mmol) was dissolved in tetrahydrofuran (25 mL), and a solution of sodium ethoxide in ethanol (3.5 g, 10 mmol, 20% w/w) was added thereto at 0°C, followed by reacting at 0°C for 1 hour. After completion of the reaction, the reaction solution was concentrated, and a mixed solution (20 mL) of 50:1 methyl tert-butyl ether and dichloromethane was added thereto. The reaction solution was concentrated, filtered and washed with 50:1 methyl tert-butyl ether and dichloromethane (10 mL×3). The obtained solid was dried under vacuum to obtain intermediate **3e** (2.0 g, yield: 99%).

MS(ESI) m/z 178.0 [M+H] $^+$

$^1$H NMR (400MHz, DMSO_*d*$_6$) δ = 7.12 (d, J = 5.6 Hz, 1H), 6.43 (d, J = 5.2 Hz, 1H), 5.29 (s, 1H).

Step 4

(S)-N-((S)-1'-(8-((3-Chloro-2-((methyl-*d*$_3$)amino)pyridin-4-yl)thio)-7-methylimidazo[ 1,2-c]pyrimidin-5-yl)-5,7-dihydrospiro[cyclopenta[b]pyridine-6,4'-piperidin]-5-yl)-2-methylpropane-2-sulfenamide **3f**

Compound **2b** (200 mg, 0.39 mmol) and intermediate **3e** (156 mg, 0.77 mmol) were dissolved in 1,4-dioxane (5 mL) under nitrogen atmosphere, and cuprous iodide (74 mg, 0.39 mmol), N,N'-dimethylethylenediamine (34 mg, 0.39 mmol) and potassium phosphate (246 mg, 1.2 mmol) were added thereto, followed by reacting under heating at 130°C under nitrogen atmosphere for 15 hours. After completion of the reaction, ammonia (30 mL) and ethyl acetate (15 mL) were added thereto. The aqueous phase was extracted with ethyl acetate (25 mL×3). All organic phases were combined,

washed with saturated aqueous sodium chloride solution, dried over anhydrous sodium sulfate and concentrated. The obtained residues were purified by silica gel chromatography with dichloromethane and methanol as eluents to obtain compound **3f** (130 mg, yield: 55%).

MS(ESI) m/z 614.3 $[M+H]^+$

$^1H$ NMR (400MHz, $CDCl_3$) δ = 8.47 (d, J=4.4 Hz, 1H), 7.68 (d, J=5.6 Hz, 1H), 7.64 (d, J=7.2 Hz, 1H), 7.48 (dd, J=1.2, 9.2 Hz, 2H), 7.17 (dd, J=4.8, 7.6 Hz, 1H), 5.73 (d, J=5.6 Hz, 1H), 5.28 (s, 1H), 5.03 (s, 1H), 4.64 (d, J=10.0 Hz, 1H), 4.05 - 3.93 (m, 2H), 3.73 (d, J=10.0 Hz, 1H), 3.33 - 3.16 (m, 3H), 2.94 (d, J=16.4 Hz, 1H), 2.54 (s, 3H), 2.52 - 2.44 (m, 1H), 2.13 (dt, J=4.0, 12.4 Hz, 1H), 1.82 - 1.74 (m, 1H), 1.52 - 1.44 (m, 1H), 1.30 (s, 9H).

Step 5

(S)-1'-(8-((3-chloro-2-((methyl-*d*$_3$)amino)pyridin-4-yl)thio)-7-methylimidazo[1,2-c]p yrimidin-5-yl)-5,7-dihydrospiro[cyclopenta[b]pyridine-6,4'-piperidin]-5-amine **3**

Compound **3f** (130 mg, 0.21 mmol) was dissolved in dry dichloromethane (4.5 mL), and a solution of hydrogen chloride in 1,4-dioxane (1.5 mL, 4 N) was added dropwise thereto at 0°C, followed by reacting at 20°C for 1 hour. 0.1 M aqueous sodium hydroxide solution (30 mL) was added to adjust pH = 14, and then the reaction solution was extracted with dichloromethane (30 mL×2). The organic phases were combined, dried over anhydrous sodium sulfate and filtered. The filtrate was concentrated under reduced pressure to obtain the crude product. The crude product was purified by reverse phase chromatography with 0.1% ammonia and acetonitrile as eluents to obtain compound **3** (65 mg, yield: 41%).

MS(ESI) m/z 510.2 $[M+H]^+$

$^1H$ NMR (400MHz, MeOD_d4) δ = 8.36 (d, J=4.4 Hz, 1H), 7.86 (d, J=7.6 Hz, 1H), 7.76 (d, J=1.6 Hz, 1H), 7.57 (d, J=5.6 Hz, 1H), 7.47 (d, J=1.6 Hz, 1H), 7.29 (dd, J=4.8, 7.6 Hz, 1H), 5.75 (d, J=6.0 Hz, 1H), 4.11 (s, 1H), 4.05 (br d, J=13.6 Hz, 2H), 3.45 - 3.35 (m, 2H), 3.27 (d, J=16.8 Hz, 1H), 2.97 (d, J=16.4 Hz, 1H), 2.55 (s, 3H), 2.17 - 2.03 (m, 2H), 1.74 (br d, J=14.0 Hz, 1H), 1.53 (br d, J=13.6 Hz, 1H).

Example 4

(S)-1'-(8-((2-(Bis(methyl-d3)amino)-3-chloropyridin-4-yl)thio)-7-methylimidazo[1,2-c]pyrimidin-5-yl)-5,7-dihydrospiro[cyclopenta[b]pyridine-6,4'-piperidin]-5-amine

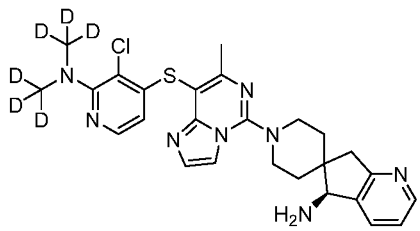

Intermediate 4a

Sodium 2-(bis(methyl-*d*$_3$)amino)-3-chloropyridine-4-thiolate

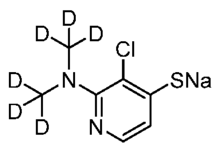

**4a**

The synthesis steps of intermediate 4a referred to those of intermediate 3e, wherein the compound methyl-*d*$_3$-amine hydrochloride was replaced by dimethyl-*d*$_6$-amine hydrochloride to prepare the aforementioned intermediate 4a.

MS(ESI) m/z 195.1 $[M+H]^+$

$^1$H NMR (400MHz, DMSO_$d_6$) $\delta$ = 7.24 (d, J = 5.2 Hz, 1H), 6.79 (d, J = 5.2 Hz, 1H).
The synthesis steps of compound 4 referred to those of Example 3, wherein intermediate **3e** was replaced by intermediate **4a** to prepare compound 4.
MS(ESI) m/z 527.2 [M+H] $^+$
$^1$H NMR (400MHz, MeOD_$d_4$) $\delta$ = 8.36 (d, J = 4.4 Hz, 1H), 7.86 (d, J = 7.6 Hz, 1H), 7.77 (d, J = 1.6 Hz, 1H), 7.72 (d, J = 5.2 Hz, 1H), 7.47 (d, J = 1.6 Hz, 1H), 7.29 (dd, J = 5.2, 7.6 Hz, 1H), 6.06 (d, J = 5.6 Hz, 1H), 4.11 (s, 1H), 4.06 (br d, J = 13.6 Hz, 2H), 3.44-3.37 (m, 2H), 3.25 (s, 1H), 2.97 (d, J = 16.8 Hz, 1H), 2.56 (s, 3H), 2.14-2.03 (m, 2H), 1.75 (br d, J = 13.2 Hz, 1H), 1.54 (br d, J = 13.6 Hz, 1H).

Example 5

(S)-1'-(8-((3-Chloro-2-((methyl-d2)amino)pyridin-4-yl)thio)-7-methylimidazo[1,2-c]p yrimidin-5-yl)-5,7-dihydrospiro[cyclopenta[b]pyridine-6,4'-piperidin]-5-amine

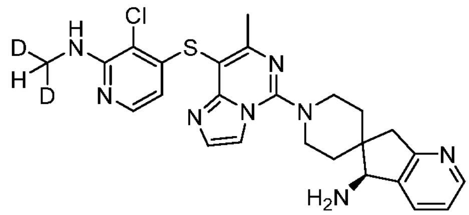

Intermediate 5f

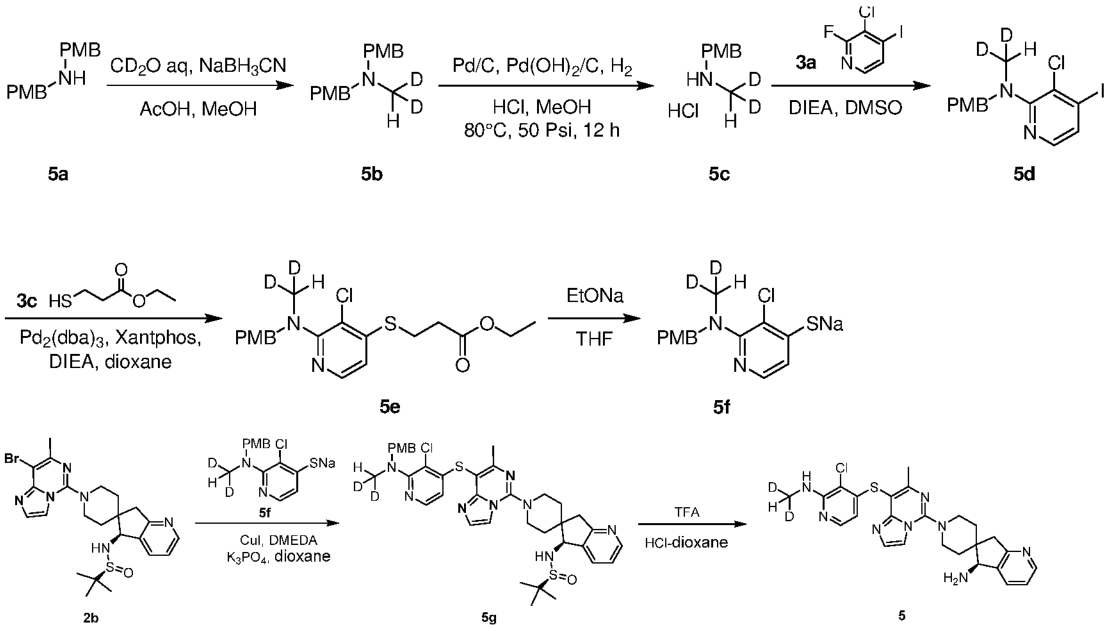


Step 1

N,N-Bis(4-methoxybenzyl)methylamine-$d_2$ **5b**

Compound **5a** (4.0 g, 16 mmol) was dissolved in methanol (50 mL), and a deuterated aqueous solution of deuterated formaldehyde (3.7 g, 23 mmol, 20% w/w) and acetic acid (0.93 g, 16 mmol) were added thereto at room temperature. Then sodium cyanoborohydride (2.9 g, 47 mmol) was added, and the reaction solution was reacted at room temperature for 15 hours. After completion of the reaction, the reaction solution was concentrated, and 2 M sodium hydroxide solution was added thereto to adjust the pH to 9-10, followed by extracting with ethyl acetate (30 mL×3). The organic phases were combined, dried over anhydrous sodium sulfate and concentrated under reduced pressure. The residues were purified by silica gel chromatography with petroleum ether and ethyl acetate as eluents to obtain compound

**5b** (4.0 g, yield: 95%).

MS(ESI) m/z 274.3 [M+H] $^+$

$^1$H NMR: (400MHz, $CDCl_3$) δ = 7.28-7.25 (m, 4H), 6.89-6.84 (m, 4H), 3.88-3.74 (m, 7H), 3.45 (s, 4H).

Step 2

N-(4-methoxybenzyl)methane-$d_2$-amine hydrochloride **5c**

Compound **5b** (1.0 g, 3.7 mmol) was dissolved in methanol (20 mL), and 10% palladium on carbon (1% water content, 100 mg), 20% palladium hydroxide (100 mg) and concentrated hydrochloric acid (0.5 mL) were added thereto. The reaction solution was reacted at 80°C under 50 psi hydrogen atmosphere for 12 hours. The reaction solution was filtered and washed with methanol (30 mL×3). The filtrate was concentrated and dried under reduced pressure to obtain compound **5c** (0.69 g, yield: 99%).

MS(ESI) m/z 153.8 [M+H] $^+$

$^1$H NMR (400MHz, DMSO-$d_6$) δ = 9.18 (br s, 2H), 7.45 (d, J = 8.4 Hz, 2H), 6.97 (d, J = 8.4 Hz, 2H), 4.01 (t, J = 5.6 Hz, 2H), 3.76 (s, 3H), 2.44 (br s, 1H).

Step 3

3-Chloro-4-iodo-N-(4-methoxybenzyl)-N-(methyl-$d_2$)pyridin-2-amine **5d**

Compound **5c** (638 mg, 3.4 mmol) and compound **3a** (787 mg, 3.1 mmol) were dissolved in DMSO (10 mL), and DIEA (2.0 g, 15 mmol) was added, followed by reacting at 60°C for 15 hours. After completion of the reaction, an ice-water mixture (100 mL) was added, and the reaction solution was extracted with ethyl acetate (30 mL×3). The organic phases were combined, washed with saturated brine, dried over anhydrous sodium sulfate and concentrated under reduced pressure. The obtained residues were purified by silica gel chromatography with petroleum ether and ethyl acetate as eluents to obtain compound **5d** (590 mg, yield: 49%).

MS(ESI) m/z 391.0 [M+H] $^+$

$^1$H NMR: (400MHz, $CDCl_3$) δ = 7.76 (d, J = 5.2 Hz, 1H), 7.35 (d, J = 5.2 Hz, 1H), 7.32-7.27 (m, 2H), 6.95-6.81 (m, 2H), 4.45 (s, 2H), 3.82 (s, 3H), 2.80 (s, 1H).

Step 4

Ethyl 3-((3-Chloro-2-((4-methoxybenzyl)(methyl-$d_2$)amino)pyridin-4-yl)thio)propionate **5e**

Compound **5d** (590 mg, 1.5 mmol) was dissolved in 1,4-dioxane (8 mL), ethyl 3-thiopropionate **3c** (304 mg, 2.3 mmol), tris(dibenzylideneacetone)dipalladium (69 mg, 0.076 mmol), 4,5-bis(diphenylphosphino)-9,9-dimethylxanthene (87 mg, 0.15 mmol) and N,N-diisopropylethylamine (586 mg, 4.5 mmol) were added thereto. The reaction solution was reacted at 100°C under nitrogen atmosphere for 5 hours. After completion of the reaction, the reaction solution was filtered. The filtrate was concentrated under reduced pressure. The obtained residues were purified by silica gel chromatography with ethyl acetate and petroleum ether as eluents to obtain compound **5e** (614 mg, yield: 94%).

MS(ESI) m/z 397.1 [M+H] $^+$

$^1$H NMR (400MHz, $CDCl_3$) δ = 8.07 (d, J = 5.6 Hz, 1H), 7.34-7.29 (m, 2H), 6.93-6.83 (m, 2H), 6.71 (d, J = 5.2 Hz, 1H), 4.43 (s, 2H), 4.20 (q, J = 7.2 Hz, 2H), 3.81 (s, 3H), 3.24 (t, J = 7.6 Hz, 2H), 2.80 (s, 1H), 2.75 (t, J = 7.6 Hz, 2H), 1.29 (t, J = 7.2 Hz, 3H).

Step 5

Sodium 3-chloro-2-((4-methoxybenzyl)(methyl-$d_2$)amino)pyridine-4-thiolate Intermediate **5f**

Compound **5e** (614 mg, 1.4 mmol) was dissolved in tetrahydrofuran (8 mL), and a solution of sodium ethoxide in ethanol (582 mg, 1.7 mmol, 20% w/w,) was added thereto at 0°C, followed by reacting at 0°C for 1 hour. After completion of the reaction, the reaction solution was concentrated, and a mixed solution of methyl tert-butyl ether and dichloromethane (6 mL, v/v = 50/2) was added thereto. The reaction solution was concentrated, filtered and washed with methyl tert-butyl ether (10 mL×3). The obtained solid was dried under reduced pressure to obtain intermediate **5f** (445 mg, yield: 98%).

MS(ESI) m/z 297.1 [M+H] $^+$

$^1$H NMR (400MHz, Methanol_$d_4$) δ = 7.46 (d, J = 5.4 Hz, 1H), 7.32-7.22 (m, 2H), 7.17 (d, J = 5.6 Hz, 1H), 6.92-6.76 (m, 2H), 4.25 (s, 2H), 3.77 (s, 3H), 2.61 (s, 1H).

Step 6

(S)-N-((S)-1'-(8-((3-Chloro-2-((4-methoxybenzyl)(methyl-*d*$_2$)amino)pyridin-4-yl)thio )-7-methylimidazo[1,2-c]pyrimidin-5-yl)-5,7-dihydrospiro[cyclopenta[b]pyridine-6,4' -piperidin]-5-yl)-2-methylpropane-2-sulfenamide **5g**

Compound **2b** (153 mg, 0.30 mmol) and intermediate **5f** (188 mg, 0.59 mmol) were dissolved in 1,4-dioxane (5 mL) under nitrogen atmosphere, and cuprous iodide (56 mg, 0.30 mmol), N,N'-dimethylethylenediamine (52 mg, 0.59 mmol) and potassium phosphate (188 mg, 0.89 mmol) were added thereto, followed by reacting under heating at 130°C under nitrogen atmosphere for 15 hours. After completion of the reaction, ammonia (20 mL) and ethyl acetate (10 mL) were added thereto. The aqueous phase was extracted with ethyl acetate (20 mL×3). All organic phases were combined, washed with saturated aqueous sodium chloride solution, dried over anhydrous sodium sulfate and concentrated. The obtained residues were purified by silica gel chromatography with dichloromethane and methanol as eluents to obtain compound **5g** (190 mg, yield: 45%).

MS(ESI) m/z 733.3 $[M+H]^+$

Step 7

(S)-1'-(8-((3 -chloro-2-((methyl-*d*$_2$)amino)pyridin-4-yl)thio)-7-methylimidazo[1,2-c]p yrimidin-5-yl)-5,7-dihydrospiro[cyclopenta[b]pyridine-6,4'-piperidin]-5-amine **5**

Compound **5g** (160 mg, 0.22 mmol) was dissolved in TFA (3 mL), followed by reacting at room temperature for 4 hours. Then a solution of hydrogen chloride in 1,4-dioxane (1 mL, 4 N) was added dropwise thereto at 0°C, followed by reacting at room temperature for 1 hour. 0.1 M aqueous sodium hydroxide solution (30 mL) was added to adjust pH = 14, and then the reaction solution was extracted with dichloromethane (30 mL×2). The organic phases were combined, dried over anhydrous sodium sulfate and filtered. The filtrate was concentrated under reduced pressure to obtain the crude product, which was purified by silica gel chromatography with dichloromethane and methanol as eluents to obtain compound 5 (51 mg, yield: 46%).

MS(ESI) m/z 509.2 $[M+H]^+$

$^1$H NMR (400MHz, $CDCl_3$) $\delta$ = 8.45 (d, J=4.4 Hz, 1H), 7.71 (d, J=5.2 Hz, 1H), 7.67 (d, J=7.2 Hz, 1H), 7.56 (d, J=1.2 Hz, 1H), 7.44 (d, J=1.6 Hz, 1H), 7.18 (dd, J=5.2, 7.6 Hz, 1H), 5.78 (d, J=5.6 Hz, 1H), 5.03 (d, J=4.4 Hz, 1H), 4.11 (s, 1H), 4.04 - 3.93 (m, 2H), 3.33 (q, J=11.6 Hz, 2H), 3.25 (d, J=16.8 Hz, 1H), 3.00 (br s, 1H), 2.93 (d, J=16.8 Hz, 1H), 2.58 (s, 3H), 2.11 (dt, J=4.0, 12.8 Hz, 1H), 2.02 (dt, J=4.4, 12.4 Hz, 1H), 1.81 - 1.73 (m, 1H), 1.52 - 1.47 (m, 1H).

Example 6

(S)-1'-(8-((3-Chloro-2-((methyl-*d*)amino)pyridin-4-yl)thio)-7-methylimidazo[1,2-c]py rimidin-5-yl)-5,7-dihydrospiro[cyclopenta[b]pyridine-6,4'-piperidin]-5-amine

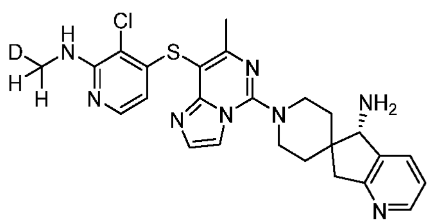

Intermediate 6f

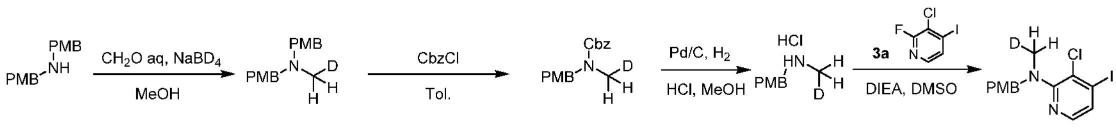


5a 6a 6b 6c 6d

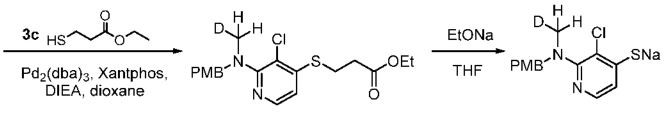


6e 6f

Intermediate 6f

N, N-bis(4-methoxybenzyl)methylamine-*d* **6a**

Compound 5a (3.0 g, 12 mmol) was dissolved in anhydrous methanol (30 mL) and aqueous formaldehyde solution (2.6 mL, 37% w/w) and acetic acid (0.67 mL, 12 mmol) were added thereto at room temperature, followed by reacting at room temperature for 2 hours. Sodium borodeuteride (0.97 g, 23 mmol) was added at 0°C, and the reaction solution was reacted at room temperature for another 1.5 hours. After completion of the reaction, the reaction solution was concentrated, Water (50 mL) was added, and the reaction solution was extracted with ethyl acetate (30 mL×3). The organic phases were combined, dried over anhydrous sodium sulfate and concentrated under reduced pressure to obtain compound **6a** (3.3 g, yield: 94%).

$^1$H NMR: (400MHz, $CDCl_3$) δ = 7.30-7.25 (m, 4H), 6.91-6.84 (m, 4H), 3.81 (s, 6H), 3.45 (s, 4H), 2.13 (s, 2H).

Step 2

Benzyl (4-methoxybenzyl)(methyl-*d*)carbamate **6b**

Compound **6a** (3.3 g, 12 mmol) was dissolved in toluene (30 mL), and benzyl chloroformate (4.1 mL, 29 mmol) was added thereto, followed by reacting 120°C under nitrogen atmosphere for 14 hours. After completion of the reaction, the reaction solution was concentrated under reduced pressure, ethyl acetate (50 mL) was added thereto, followed by washing water (20 mL) and saturated brine (20 mL×2). The organic phase was dried over anhydrous sodium sulfate and concentrated under reduced pressure. The residues were purified by silica gel chromatography with petroleum ether and ethyl acetate as eluents to obtain compound **6b** (3.8 g crude product).

$^1$H NMR (400MHz, $CDCl_3$) δ = 7.43-7.31 (m, 5H), 7.21 (d, J = 7.6 Hz, 1H), 7.13 (d, J = 7.6 Hz, 1H), 6.91-6.82 (m, 2H), 5.20 (s, 2H), 4.45 (s, 2H), 3.82 (s, 3H), 2.86 (d, J = 10.8 Hz, 2H).

Step 3

N-(4-Methoxybenzyl)methane-*d*-amine hydrochloride **6c**

Compound **6b** (3.8 g, 12 mmol) was dissolved in methanol (40 mL) and 10% palladium on carbon (1.0 g) was added, followed by reacting at 40°C under hydrogen atmosphere for 16 hours. The reaction solution was filtered and washed with methanol (80 mL). The filtrate was concentrated and dried under reduced pressure to obtain compound **6c** (2 g, yield: 99%).

MS(ESI) m/z 152.9 [M+H] $^+$

Step 4

3-Chloro-4-iodo-N-(4-methoxybenzyl)-N-(methyl-*d*)pyridin-2-amine **6d**

Compound **6c** (2 g, 12 mmol) and compound **3a** (2.3 g, 8.8 mmol) were dissolved in DMSO (4 mL), and DIEA (4.3 mL, 26 mmol) was added thereto, followed by stirring at 60°C for 5 hours. After completion of the reaction, water (50 mL) was added, and the reaction solution was extracted with ethyl acetate (30 mL×3). The organic phases were combined, washed with saturated brine, dried over anhydrous sodium sulfate and concentrated under reduced pressure.

The obtained residues were purified by silica gel chromatography with petroleum ether and ethyl acetate as eluents to obtain compound **6d** (2.6 g, yield: 76%).

MS(ESI) m/z 390.0 [M+H] $^+$

$^1$H NMR: (400MHz, $CDCl_3$) δ = 7.76 (d, J = 4.8 Hz, 1H), 7.35 (d, J = 5.2 Hz, 1H), 7.32-7.27 (m, 2H), 6.92-6.83 (m, 2H), 4.45 (s, 2H), 3.81 (s, 3H), 2.82 (s, 2H).

Step 5

Ethyl 3-((3-chloro-2-((4-methoxybenzyl)(methyl-*d*)amino)pyridin-4-yl)thio)propionate **6e**

Compound **6d** (2.6 g, 6.7 mmol) was dissolved in 1,4-dioxane (30 mL), and ethyl 3-thiopropionate **3c** (1.3 g, 10 mmol), tris(dibenzylideneacetone)dipalladium (310 mg, 0.34 mmol), 4,5-bis(diphenylphosphino)-9,9-dimethylxanthene (390 mg, 0.67 mmol) and N,N-diisopropylethylamine (3.3 mL, 20 mmol) were added thereto, followed by stirring at 100°C under nitrogen atmosphere for 6 hours. After completion of the reaction, the reaction solution was filtered. The filtrate was concentrated under reduced pressure. The obtained residues were purified by silica gel chromatography with ethyl acetate and petroleum ether as eluents to obtain compound **6e** (2.3 g, yield: 88%).

MS(ESI) m/z 396.1 [M+H] $^+$

$^1$H NMR (400MHz, $CDCl_3$) δ = 8.07 (d, J = 5.2 Hz, 1H), 7.34-7.28 (m, 2H), 6.93-6.82 (m, 2H), 6.72 (d, J = 5.2 Hz, 1H), 4.43 (s, 2H), 4.20 (q, J = 7.2 Hz, 2H), 3.81 (s, 3H), 3.24 (t, J = 7.6 Hz, 2H), 2.81 (s, 2H), 2.75 (t, J = 7.6 Hz, 2H), 1.29 (t, J = 6.8 Hz, 3H).

Step 6

Sodium 3-chloro-2-((4-methoxybenzyl)(methyl-*d*)amino)pyridine-4-thiolate Intermediate **6f**

Compound **6e** (2.3 g, 5.9 mmol) was dissolved in tetrahydrofuran (25 mL), and a solution of sodium ethoxide in ethanol (2.4 g, 7.1 mmol, 20% w/w) at 0°C, followed by stirring at 0°C for 1 hour. After completion of the reaction, the reaction solution was concentrated. A mixed solution of methyl tert-butyl ether and dichloromethane (40 mL, v/v = 50/2) was added, and the reaction solution was filtered, and washed with methyl tert-butyl ether (15 mL×3). The obtained solid was dried under vacuum to obtain intermediate **6f** (1.7 g, yield: 91%).

$^1$H NMR (400MHz, DMSO-d6) δ = 7.29-7.23 (m, 3H), 6.89-6.79 (m, 3H), 4.12 (s, 2H), 3.72 (s, 3H), 3.34 (s, 2H).

The synthesis steps of compound 6 referred to Example **5,** wherein intermediate **5f** was replaced by intermediate **6f** to prepare the aforementioned compound 6.

MS(ESI) m/z 508.2 [M+H] $^+$

$^1$H NMR (400MHz, $CDCl_3$) δ = 8.47 (d, J=4.4 Hz, 1H), 7.68 (d, J=5.6 Hz, 1H), 7.64 (d, J=7.2 Hz, 1H), 7.48 (dd, J=1.2, 9.2 Hz, 2H), 7.17 (dd, J=4.8, 7.6 Hz, 1H), 5.73 (d, J=5.6 Hz, 1H), 5.28 (s, 1H), 5.03 (s, 1H), 4.64 (d, J=10.0 Hz, 1H), 4.05 - 3.93 (m, 2H), 3.73 (d, J=10.0 Hz, 1H), 3.33 - 3.16 (m, 3H), 2.94 (d, J=16.4 Hz, 1H), 2.54 (s, 3H), 2.52 - 2.44 (m, 1H), 2.13 (dt, J=4.0, 12.4 Hz, 1H), 1.82 - 1.74 (m, 1H), 1.52 - 1.44 (m, 1H), 1.30 (s, 9H).

## BIOLOGICALASSAY

The present disclosure will be further described with reference to the following test examples, but the examples should not be considered as limiting the scope of the present disclosure.

Test Example 1. Determination of the activity of the compounds of the present disclosure on SHP2 phosphatase

1. Experimental materials and instruments

| Name of instrument | Manufacturer | Model |
|---|---|---|
| Constant temperature shaker | IMB | MB-1002A |
| Microplate reader | MDSpectraMax | M5 |
| | | |
| Reagent name | Supplier | Cat. No. |
| Shp2 | GenScript | N/A |

(continued)

| Name of instrument | Manufacturer | Model |
|---|---|---|
| Activating polypeptide | GenScript | N/A |
| DMSO | Sigma | C34557 |
| 1M HEPES | Thermofisher | 15630080 |
| 5M NaCl | Thermofisher | AM9760G |
| 2M KCl | Thermofisher | AM9640G |
| 1M DTT | Thermofisher | P2325 |
| 10% SDS | Thermofisher | AM9822 |
| 30% Brij$^{TM}$-35 | Thermofisher | 20150 |
| EDTA | Sigma | EDS-500G |
| Difmup | Invitrogen | TM 6567 |

2. Experimental steps

0.2 nM recombinantly expressed full-length SHP2 (aa 1-593), 0.5 nM activated polypeptide IRS1 with dual phosphorylation sites (sequence: H2N-LN(pY)IDLDLY(dPEG8)LST(pY)ASINFQK-amide), and a series of concentrations of the test compound (final concentrations of 1 μM, 0.3 μM, 0.1 μM, 0.03 μM, 0.01 μM, 0.003 μM, 0.001 μM, 0.0003 μM, 0.0001 μM and 0.00003 μM) were added into the phosphatase reaction solution (60 mM HEPES, PH 7.5 0.005% Brij-35, 75 mM NaCl, 75 mM KCl, 1 mM EDTA, 5 mM DTT), followed by shaking (350 rpm) at room temperature for 30 minutes.Then the reaction substrate DiFMUP was added at a final concentration of 30 μM, and the reaction solution was reacted at room temperature for 30 minutes. Then the phosphatase reaction was stopped by adding 5 μL of the reaction stop solution (60 mM HEPES, pH 7.5, 0.2% SDS). Ex358nm/Em455 fluorescence value was read on the fluorescence plate reader MD SpectraMax.

The $IC_{50}$ value of compounds was calculated by using the four-parameter logit method. In the following equation, x represents the logarithmic form of the compound concentration; F(x) represents the effect value (the inhibition rate of cell proliferation at the given concentration): F(x) = ((A-D)/ (1+ ((x/C) ^B))) + D. A, B, C and D are four parameters. Different concentrations correspond to different inhibition rates of phosphatase activity. A reverse curve was plotted and the $IC_{50}$ of the inhibitors was calculated from the curve. The $IC_{50}$ of compounds was calculated by using Primer premier 6.0.

The *in vitro* activity of the compound of the present disclosure on SHP2 was determined by the above assay. The oral active SHP2 inhibitor SHP099 was selected as the positive drug, the structure of which was disclosed in the document J. Med. Chem. 2016, 59, 7773-7782, and this particular compound was purchased from Shanghai Haoyuan Chemexpress Co., Ltd. (Medchemexpress.cn).

The measured $IC_{50}$ values are shown in Table 1.

Table 1. $IC_{50}$ of the compounds of the present disclosure on SHP2 phosphatase

| Example No. | $IC_{50}$ (nM) | Example No. | $IC_{50}$ (nM) |
|---|---|---|---|
| SHP099 | 79 | 1 | 1.7 |
| 2 | 2.1 | 3 | 4.5 |
| 5 | 4.8 | 6 | 4.7 |

Test Example 2. *In vitro* metabolic stability experiment in rat liver microsomes

The concentration of the compound in the reaction system was determined by LC/MS/MS, so as to calculate the intrinsic clearance rate of the test compound and to evaluate the *in vitro* metabolic stability in rat liver microsomes.

222.5 μL, 1.1236 mg/mL of a mixed solution of rat liver microsomes (male Wistar Han strain, purchased from Corning, catalog number 452511) and 25 μL, 10 mM of NADPH were added into the incubation plate. The mixture was mixed by vortexing for 10 seconds. The plate was incubated in a water bath at 37°C for 8 minutes. 2.5 μL, 100 μM of the test compound or positive control was added to the incubation plate to start the reaction. The mixture was mixed by vortexing for 12 seconds, and incubated in a water bath at 37°C. The reaction was stopped by transferring 20 μL of the

incubation system to a stop plate containing 100 μL of cold stop solution at 0.5, 5, 10, 15, 20 and 30 minutes. The mixture was mixed by vortexing for 2 minutes. The stop plate was centrifuged at 4000 rpm for 20 minutes, then left to stand at 4°C for 30 minutes, and then centrifuged at 4000 rpm for 20 minutes. 40 μL of the supernatant of each compound was transferred to a 96-well injection plate, and 160 μL of pure water was added to dilute the samples.

The obtained samples were quantified by ion chromatogram, and the residual rate was calculated according to the peak area of the test compounds or positive control. The slope k was determined by linear regression of the natural logarithm of the residual rate to the incubation time by using Microsoft Excel.

The *in vitro* half-life (in vitro t1/2) was calculated from the slope: in vitro t1/2 = -(0.693/k)

The following equation was used to convert *in vitro* half-life into intrinsic clearance rate (in vitro CLint, μL/min/mg protein):

$$\text{in vitro CLint} = (0.693/t1/2) \times (\text{incubation volume } (\mu L)/\text{protein amount (mg)})$$

The measured values of intrinsic clearance rate in rat liver microsomes are shown in Table 2.

Table 2. Intrinsic clearance rate of the compounds of the present invention in rat liver microsomes

| Example No. | Intrinsic clearance rate (μL/min/mg protein) |
|---|---|
| 2 | 19.2 |
| 3 | <3 |
| 5 | 26.62 |
| 6 | 34.67 |

Test Example 3. *In vivo* pharmacokinetic experiments in rats

Rats were used as the test animals. The plasma drug concentration at different times after the rats were given the compounds of the invention by gavage was determined by using the LC/MS/MS method. The *in vivo* pharmacokinetic behavior of the compounds of the present invention in rats was studied, and pharmacokinetic characteristics thereof were evaluated.

Test animals: 3 healthy, 6-8 weeks old male SD rats in each group

Drug formulation

A certain amount of drug was weighed, and 0.5% by mass of hypromellose, 0.1% by volume of Tween 80 and 99.4% by volume of water were added thereto to formulate a white suspension at 1 mg/mL.

Drug administration

SD rats were fasted overnight, then the drug was administered by gavage. The dosage of reference 1 was 7.5 mg/kg, and the dosage of Example 1 was 5 mg/kg.

Operation

Rats were administered the compounds of the present invention by gavage. At 0.25, 0.5, 1, 2, 4, 8 and 24 hours after administration, 0.2 mL of blood was collected from the jugular vein and placed in a test tube containing EDTA-K2. The tube was centrifuged at 4°C, 4000 rpm for 5 minutes to separate the plasma, which was stored at -75°C.

Determination of the content of the test compounds in rat plasma after administration of different concentrations of drugs by gavage: 50 μL of rat plasma at each time after administration was taken, and 200 μL of a solution of the internal standard dexamethasone in acetonitrile (50 ng/mL). The mixture was mixed by vortexing for 30 seconds, centrifuged at 4°C, 4700 rpm for 15 minutes. The supernatant of the plasma samples were collected and diluted three times with water, and 2.0 μL was taken for the LC/MS/MS analysis.

Results of pharmacokinetic parameters

The rat pharmacokinetic parameters of the compounds of the present invention are shown in Table 3 below.

Table 3. Rat pharmacokinetic parameters of the compounds of the invention

| No. | | Maximum blood conc. | Area under curve | Area under curve of unit dose | Metabolite ratio |
|---|---|---|---|---|---|
| | | Cmax (ng /mL) | AUC (ng /mL*h) | AUC/D (mg/mL*h) | $AUC_{metabolite}$/ $AUC_{original}$ drug |
| 2 | 21 (7.5 mpk) | 500 | 4250 | 567 | 0.11 |
| | Metabolite 14 | 48.4 | 456 | 60.8 | |
| 3 | 21 (5 mpk) | 445 | 3819 | 764 | 0.071 |
| | Metabolite 14 | 26.7 | 264 | 54.5 | |

Test Example 4. *In vivo* pharmacokinetic experiments in cynomolgus monkeys

Cynomolgus monkeys were used as the test animals. The plasma drug concentration at different times after the cynomolgus monkeys were given the compounds of the invention by gavage was determined by using the LC/MS/MS method. The *in vivo* pharmacokinetic behavior of the compounds of the present invention in cynomolgus monkeys was studied, and pharmacokinetic characteristics thereof were evaluated.

Test animals: 3 healthy, 2-5 years old male cynomolgus monkeys in each group;

Drug formulation

Administration by gavage: a certain amount of drug was weighed, and 0.5% by mass of hypromellose, 0.1% by volume of Tween 80 and 99.4% by volume of water were added thereto to formulate a white suspension at 1 mg/mL.

Drug administration

The cynomolgus monkeys were fasted overnight, and then the drug was administered by gavage at a dose of 5 mg/kg.

Operation

Cynomolgus monkeys were administered the compounds of the present invention by gavage. At 0.25, 0.5, 1, 2, 4, 8 and 24 hours after administration, 0.2 mL of blood was collected from the peripheral vein and placed in a test tube containing EDTA-K2. The tube was centrifuged at 2 to 8°C, 2000 rpm for 10 minutes to separate the plasma, which was stored at -75°C.

Determination of the content of the test compounds in cynomolgus monkey plasma after administration of different concentrations of drugs by gavage: 55 μL of cynomolgus monkey plasma at each time after administration was taken, and 200 μL of a solution of the internal standard verapamil or dexamethasone in acetonitrile. The mixture was mixed by vortexing for 30 seconds, centrifuged at 4°C, 3900 rpm for 15 minutes. The supernatant of the plasma samples were collected and diluted three times with water, and 15 μL was taken for LC/MS/MS analysis.

Results of pharmacokinetic parameters

The cynomolgus monkey pharmacokinetic parameters of the compounds of the present invention are shown in Table 4 below.

Table 4. Cynomolgus monkey pharmacokinetic parameters of the compounds of the invention

| No. | | Maximum blood conc. | Area under curve | Metabolite ratio |
|---|---|---|---|---|
| | | Cmax (ng /mL) | AUC (ng /mL*h) | $AUC_{metabolite}$/ $AUC_{original}$ drug |
| 2 | 2 (5 mpk) | 12677 | 70987 | 0.39 |
| | Metabolite 14 | 3647 | 27920 | |
| 3 | 2 (5 mpk) | 12200 | 72303 | 0.20 |
| | Metabolite 14 | 1400 | 14235 | |

Test example 5. Permeability test in Caco-2

The apparent permeability coefficient ($P_{app}$) of the analyzed drugs was determined by liquid chromatography tandem mass spectrometry (LC/MS/MS), using the Caco-2 cell model.

210 μL of HBSS (25 mM HEPES, pH 7.4, containing 50 μM quinidine, 30 μM benzbromarone and 20 μM sulfasalazine) containing 10 μM test compound was added to the top of Transwell (purchased from Corning) chamber with Caco-2 cells (purchased from ATCC) at a density of 7.92×10□ cells/cm$^2$. At the same time, 10 μL of the sample was immediately taken to a deep 96-well plate with 90 μL of HBSS (25 mM HEPES, pH 7.4, containing 50 μM quinidine, 30 μM benzbromarone and 20 μM sulfasalazine) as the initial sample of the dosing end. 800 μL of HBSS (25 mM HEPES, pH 7.4, containing 50 μM quinidine, 30 μM benzbromarone and 20 μM sulfasalazine) was added to the basal end. The cells were incubated at 37°C for 2 hours. At the time points of 45 minutes and 2 hours, 10 μL of the sample from the top was respectively pipetted into the deep-well 96-well plate containing 90 μL of HBSS (25 mM HEPES, pH 7.4, containing 50 μM quinidine, 30 μM benzbromarone and 20 μM sulfasalazine). At the time points of 45 minutes and 2 hours, 100 μL of the sample from the basal end was respectively pipetted into the deep-well 96-well plate. Then 3 times the volume of pre-cooled internal standard was added to each well. The mixture was vortexed at 1000 rpm for 10 minutes and centrifuged at 4000 rpm for 20 minutes. 100 μL of the sample was taken from each well, and the 3 samples were mixed with 100 μL of pure water for LC/MS/MS analysis.

Data was calculated by using Microsoft Excel, and the peak area was calculated based on the chromatograms. The unit of apparent permeability coefficient (Papp) is cm/s, calculated by using the following equation:

$$P_{app} = \frac{C_R^{120} \times 0.8 - C_R^{45} \times 0.7}{(C_D^{45} + C_D^{120})/2 \times Area \times time}$$

$C_R$ is the concentration of the test compound at the basal end (superscript "120" or "45" is the sampling time, unit: minutes), $C_D$ is the concentration of the test compound at the top (superscript "120" or "45" is the sampling time, unit: minutes), Area is the surface area of the membrane (0.33 cm$^2$), and time is the total operation time (75×60 seconds).

The measured apparent permeability coefficient values in Caco-2 cells are shown in Table 5.

Table 5. Apparent permeability coefficient of the compounds of the present invention in Caco-2 cells

| Example No. | Papp $_{(A-B)}$ ($10^{-6}$, cm/s) |
|---|---|
| 1 | 2.71 |
| 2 | 12.05 |
| 3 | 17.22 |

Test Example 6. CYP inhibition experiment

Mixed human liver microsomes from 150 donors (purchased from Corning, catalog number 452117) were used to evaluate the representative substrate metabolic responses of the five main human CYP subtypes (CYP1A2, CYP2C9, CYP2C19, CYP2D6 and CYP3A4/5). The influence of different concentrations of test compounds on the metabolic responses of phenacetin (CYP1A2), diclofenac sodium (CYP2C9), S-mephenytoin (CYP2C19), bufurolol hydrochloride (2D6) and midazolam (CYP3A4/5) was determined by liquid chromatography tandem mass spectrometry (LC/MS/MS).

200 μL of the reaction system (100 mmol/L phosphate buffer, pH 7.4, containing 0.3% DMSO, 0.6% acetonitrile and 0.1% methanol by volume respectively) with 30 μM phenacetin, 10 μM diclofenac sodium, 35 μM S-mephenytoin,

5 μM bufurolol hydrochloride, 3 μM midazolam, 1 mM NADPH, test compound (at a concentration of 0.1, 0.3, 1, 3, 10 and 30 μmol/L respectively) or positive compound or blank control and mixed human liver microsomes (0.2 mg/mL) was incubated at 37°C for 5 minutes. Then 200 μL of a solution containing 3% formic acid and 40 nM of the internal standard verapamil in acetonitrile was added, and the reaction system was centrifuged at 4000 rpm for 50 minutes. The reaction system was placed on ice to cool for 20 minutes, and then centrifuged at 4000 rpm for 20 minutes to separate the protein. 200 μL of the supernatant was taken for LC/MS/MS analysis.

The peak area was calculated based on the chromatograms. The residual activity ratio (%) was calculated using the following equation:

$$\text{Peak area ratio} = \text{metabolite peak area} / \text{internal standard peak area}$$

$$\text{Residual activity ratio (\%)} = \text{peak area ratio of the test compound group} / \text{peak area ratio of the blank group}$$

The CYP median inhibitory concentration ($IC_{50}$) was calculated by Excel XLfit 5.3.1.3.

The measured CYP median inhibitory concentration ($IC_{50}$) values are shown in Table 6.

Table 6. Median inhibitory concentration ($IC_{50}$) of the compounds of the present invention on CYP

| Example No. | CYP 1A2 (μM) | CYP2C9 (μM) | CYP 2C19 (μM) | CYP2D6 (μM) | CYP 3A4/5 (μM) |
|---|---|---|---|---|---|
| 1 | 9.21 | >20 | >20 | >20 | >20 |
| 3 | 14.7 | >20 | >20 | >20 | >20 |

## Claims

**1.** A compound of general formula (II) or a tautomer, mesomer, racemate, enantiomer, diastereomer, atropisomer thereof, or mixture form thereof, or a pharmaceutically acceptable salt thereof, wherein

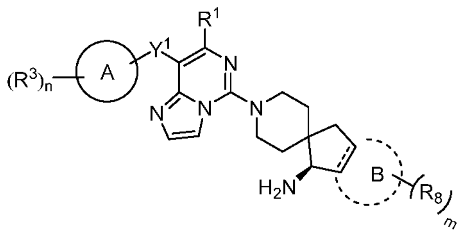

(II)

$R^1$ is selected from the group consisting of hydrogen atom, $C_{1-6}$ alkyl, halo$C_{1-6}$ alkyl and amino, the alkyl and haloalkyl are each independently optionally further substituted by one or more substituents of deuterium atom;
$Y^1$ is -S- or a direct bond;
ring A is selected from the group consisting of aryl and heteroaryl, preferably phenyl and pyridyl;
each $R^3$ is independently selected from the group consisting of hydrogen atom, deuterium atom, halogen, cyano, $C_{1-6}$ alkyl, $C_{1-6}$ alkoxy, halo$C_{1-6}$ alkyl, halo$C_{1-6}$ alkoxy, $C_{3-8}$ cycloalkyl, 3-12 membered heterocyclyl, $-OR^a$, $-CHR^aR^b$ and $-NR^aR^b$;
the $R^a$ and $R^b$ are each independently selected from the group consisting of hydrogen, deuterium atom, hydroxy, $C_{1-6}$ alkyl, 3-12 membered heterocyclyl and $C_{3-8}$ cycloalkyl, wherein the alkyl, heterocyclyl or cycloalkyl is further substituted by one or more substituents selected from the group consisting of halogen, deuterium atom, cyano, amino and hydroxy;
or $R^a$ and $R^b$ together with the atom to which they are attached form a 3-12 membered heterocyclyl or $C_{3-8}$ cycloalkyl, the alkyl, heterocyclyl or cycloalkyl is optionally further substituted by one or more substituents selected from the group consisting of halogen, deuterium atom, cyano, amino and hydroxy;
ring B is a 6-membered aromatic ring, 5-membered heteroaromatic ring or 6-membered heteroaromatic ring, preferably benzene ring or pyridine ring;

each $R^8$ is independently selected from the group consisting of hydrogen atom, deuterium atom, halogen, cyano, $C_{1-6}$ alkyl and $C_{1-6}$ alkoxy;
m is selected from the group consisting of 0, 1, 2, 3 and 4;
n is selected from the group consisting of 1, 2, 3 and 4.

2. The compound of general formula (II) or the tautomer, mesomer, racemate, enantiomer, diastereomer, atropisomer thereof, or mixture form thereof, or the pharmaceutically acceptable salt thereof according to claim 1, wherein $R^1$ is selected from the group consisting of $C_{1-6}$ alkyl and halo$C_{1-6}$ alkyl, the $C_{1-6}$ alkyl or halo$C_{1-6}$ alkyl is optionally substituted by one or more deuterium atoms.

3. The compound of general formula (II) or the tautomer, mesomer, racemate, enantiomer, diastereomer, atropisomer thereof, or mixture form thereof, or the pharmaceutically acceptable salt thereof according to claim 2, wherein $Y^1$ is -S-.

4. The compound of general formula (II) or the tautomer, mesomer, racemate, enantiomer, diastereomer, atropisomer thereof, or mixture form thereof, or the pharmaceutically acceptable salt thereof according to claim 3, wherein ring A is selected from the group consisting of phenyl and pyridyl.

5. The compound of general formula (II) or the tautomer, mesomer, racemate, enantiomer, diastereomer, atropisomer thereof, or mixture form thereof, or the pharmaceutically acceptable salt thereof according to claim 4, wherein the each $R^3$ is independently selected from the group consisting of hydrogen atom, deuterium atom, halogen, cyano, $C_{1-6}$ alkyl, $C_{1-6}$ alkoxy, halo$C_{1-6}$ alkyl, halo$C_{1-6}$ alkoxy, $C_{3-8}$ cycloalkyl, 3-12 membered heterocyclyl and $-NR^aR^b$; the $R^a$ and $R^b$ are each independently selected from the group consisting of hydrogen, deuterium atom, hydroxy and $C_{1-6}$ alkyl, the alkyl is substituted by one or more deuterium atoms.

6. The compound of general formula (II) or the tautomer, mesomer, racemate, enantiomer, diastereomer, atropisomer thereof, or mixture form thereof, or the pharmaceutically acceptable salt thereof according to claim 5, wherein ring B is a benzene ring or pyridine ring.

7. The compound of general formula (II) or the tautomer, mesomer, racemate, enantiomer, diastereomer, atropisomer thereof, or mixture form thereof, or the pharmaceutically acceptable salt thereof according to any one of claims 1-6, wherein

$R^1$ is selected from methyl, the methyl is optionally substituted by one or more deuterium atoms;
$Y^1$ is -S-;
ring A is selected from the group consisting of phenyl and pyridyl;
each $R^3$ is independently selected from the group consisting of hydrogen atom, deuterium atom, halogen and $-NR^aR^b$;
the $R^a$ and $R^b$ are each independently selected from the group consisting of hydrogen, deuterium atom and $C_{1-6}$ alkyl, the alkyl is substituted by one or more deuterium atoms;
ring B is a benzene ring or pyridine ring;
m is selected from 0;
n is selected from the group consisting of 1, 2, 3 and 4.

8. The compound of general formula (II) or the tautomer, mesomer, racemate, enantiomer, diastereomer, atropisomer thereof, or mixture form thereof, or the pharmaceutically acceptable salt thereof according to claim 7,

wherein $R^1$ is selected from methyl;
$Y^1$ is -S-;
ring A is selected from pyridyl;
each $R^3$ is independently selected from the group consisting of hydrogen atom, deuterium atom, halogen and $-NR^aR^b$;
the $R^a$ and $R^b$ are each independently selected from the group consisting of hydrogen, deuterium atom and $C_{1-6}$ alkyl, the alkyl is substituted by one or more deuterium atoms;
ring B is a pyridine ring;
m is selected from 0;
n is selected from the group consisting of 1, 2, 3 and 4.

9. The compound of general formula (II) or the tautomer, mesomer, racemate, enantiomer, diastereomer, atropisomer

thereof, or mixture form thereof, or the pharmaceutically acceptable salt thereof according to claim 7,

wherein $R^1$ is selected from methyl;
$Y^1$ is -S-;
ring A is selected from pyridyl;
each $R^3$ is independently selected from the group consisting of hydrogen atom, deuterium atom, chlorine atom, $-NH-CH_3$ and $N-(CH_3)_2$; the hydrogen atom on the methyl of the $-NH-CH_3$ or $N-(CH_3)_2$ is substituted by one or more deuterium atoms;
ring B is a pyridine ring;
m is selected from 0;
n is selected from the group consisting of 1, 2, 3 and 4.

**10.** The compound of general formula (II) or the tautomer, mesomer, racemate, enantiomer, diastereomer, atropisomer thereof, or mixture form thereof, or the pharmaceutically acceptable salt thereof according to any one of claims 1-9, being

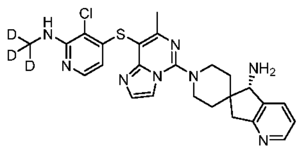 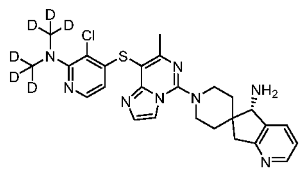 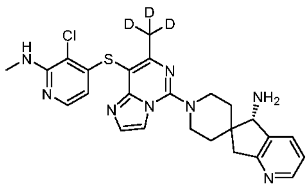

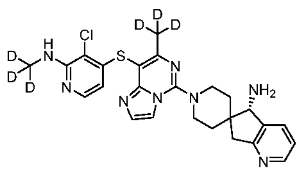 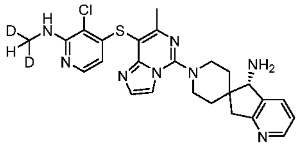 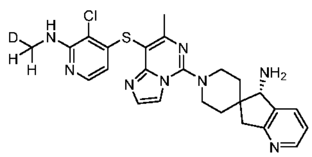

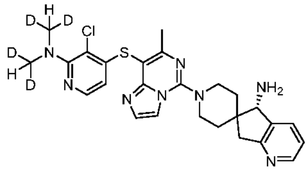 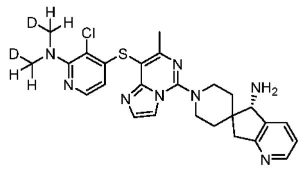 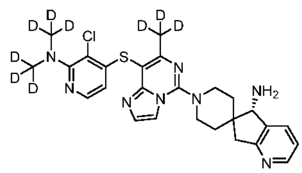

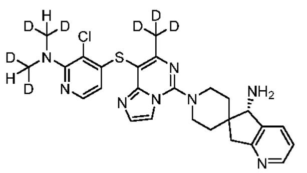 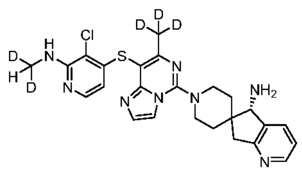

.

**11.** A preparation method of the compound of general formula (II) or the tautomer, mesomer, racemate, enantiomer, diastereomer, atropisomer thereof, or mixture form thereof, or the pharmaceutically acceptable salt thereof according to any one of claims 1-10, comprising the following step of removing the protecting group PG from a compound of formula (II-2) to obtain the compound of formula (II-1),

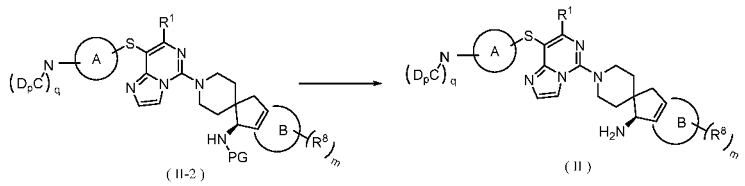

( II-2 ) ( II )

wherein, PG is an amino protecting group selected from the group consisting of Boc, PMB, $S(=O)^tBu$ and Cbz;

p is selected from the group consisting of 1, 2 and 3;
q is selected from the group consisting of 1 and 2;
wherein, ring A, ring B, $R^1$, $R^8$, B and m are as defined in claims 1-10.

**12.** The preparation method according to claim 11, further comprising the following step of,

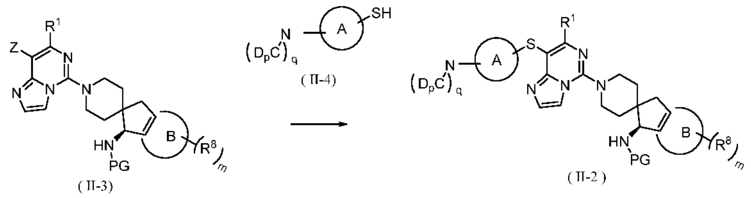

subjecting a compound of formula (II-3) and a compound of formula (II-4) to C-S coupling under alkaline conditions to obtain the compound of formula (II-2).

**13.** A pharmaceutical composition comprising 0.1-2000 mg of the compound of general formula (II) or the tautomer, mesomer, racemate, enantiomer, diastereomer, atropisomer thereof, or mixture form thereof, or the pharmaceutically acceptable salt thereof according to any one of claims 1-10, and one or more pharmaceutically acceptable carriers, diluents or excipients.

**14.** Use of the compound of general formula (II) or the tautomer, mesomer, racemate, enantiomer, diastereomer, atropisomer thereof, or mixture form thereof, or the pharmaceutically acceptable salt thereof according to any one of claims 1-10, or the pharmaceutical composition according to claim 13, in the preparation of a medicament for preventing or treating a disease or disorder mediated by SHP2 activity.

**15.** Use of the compound of general formula (II) or the tautomer, mesomer, racemate, enantiomer, diastereomer, atropisomer thereof, or mixture form thereof, or the pharmaceutically acceptable salt thereof according to any one of claims 1-10, or the pharmaceutical composition according to claim 13, as a SHP2 inhibitor in the preparation of a medicament for preventing and/or treating tumor or cancer.

**16.** Use of the compound of general formula (II) or the tautomer, mesomer, racemate, enantiomer, diastereomer, atropisomer thereof, or mixture form thereof, or the pharmaceutically acceptable salt thereof according to any one of claims 1-10, or the pharmaceutical composition according to claim 13, in the preparation of a medicament for preventing and/or treating Noonan syndrome, Leopard syndrome, juvenile myelomonocytic leukemia, neuroblastoma, melanoma, acute myeloid leukemia, breast cancer, esophageal cancer, lung cancer, colon cancer, head cancer, pancreatic cancer, head and neck squamous cell carcinoma, stomach cancer, liver cancer, anaplastic large cell lymphoma or glioblastoma.

**INTERNATIONAL SEARCH REPORT**

International application No.
**PCT/CN2020/098474**

**A. CLASSIFICATION OF SUBJECT MATTER**

A61K 31/495(2006.01)i; C07D 471/04(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

A61K,C07D,A61P

Documentation searched other than minimum documentation to the extent that such documents are included in the fields searched

Electronic data base consulted during the international search (name of data base and, where practicable, search terms used)

DWPI, SIPOABS, CNABS, CNMED, CNTXT, CNKI, REGISTRY, CAPLUS, MEDLINE, VEN, ISI-WEB OF SCIENCE, PubMed, SHP2, Src同源2蛋白质酪氨酸磷酸酶2, 蛋白酪氨酸磷酸酶(PTP), Noonan's syndrome, Moynahan syndrome, neuroblastoma, 努南综合征, 豹皮综合征, 幼年性骨髓单核细胞白血病, 神经母细胞瘤

**C. DOCUMENTS CONSIDERED TO BE RELEVANT**

| Category* | Citation of document, with indication, where appropriate, of the relevant passages | Relevant to claim No. |
|---|---|---|
| A | CN 105142634 A (INDIANA UNIVERSITY RESEARCH AND TECHNOLOGY CORPORATION) 09 December 2015 (2015-12-09)<br>claims 1-20 | 1-16 |
| A | WO 2018172984 A1 (JACOBIO PHARMACEUTICALS CO., LTD.) 27 September 2018 (2018-09-27)<br>the abstract, and description, pages 3-22, formula I and formula II | 1-16 |
| A | WO 2018081091 A1 (RELAY THERAPEUTICS, INC.) 03 May 2018 (2018-05-03)<br>the abstract, and description, pages 32-42 | 1-16 |
| A | WO 2018136265 A1 (REVOLUTION MEDICINES, INC.) 26 July 2018 (2018-07-26)<br>the abstract, and description, paragraph [0427], the compound in the table | 1-16 |
| A | CN 109311848 A (BEIJING JACOBIO PHARMACEUTICALS CO., LTD.) 05 February 2019 (2019-02-05)<br>the abstract, and description, pages 28-40 | 1-16 |

☐ Further documents are listed in the continuation of Box C.

☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **20 September 2020** | **12 October 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/098474**

| Patent document cited in search report | Publication date (day/month/year) | Patent family member(s) | Publication date (day/month/year) |
|---|---|---|---|
| CN 105142634 A | 09 December 2015 | EP 2988741 A4 | 21 December 2016 |
| | | CN 105142634 B | 12 June 2020 |
| | | US 9522881 B2 | 20 December 2016 |
| | | EP 2988741 B1 | 27 November 2019 |
| | | WO 2014176488 A1 | 30 October 2014 |
| | | AU 2014256984 A1 | 22 October 2015 |
| | | CN 110423212 A | 08 November 2019 |
| | | EP 2988741 A1 | 02 March 2016 |
| | | US 2016102054 A1 | 14 April 2016 |
| | | AU 2014256984B B2 | 14 February 2019 |
| | | CA 2944198 A1 | 30 October 2014 |
| WO 2018172984 A1 | 27 September 2018 | EP 3601239 A1 | 05 February 2020 |
| | | KR 20190140931 A | 20 December 2019 |
| | | EP 3601239 A4 | 13 May 2020 |
| | | JP 2020515538 A | 28 May 2020 |
| | | IN 201947042842 A | 25 October 2019 |
| | | TW I664175 B | 01 July 2019 |
| | | AU 2018239542 A1 | 14 November 2019 |
| | | CA 3057582 A1 | 27 September 2018 |
| | | SG 11201908820 A1 | 30 October 2019 |
| | | VN 69294 A | 25 March 2020 |
| | | TW 201840553 A | 16 November 2018 |
| | | PH 12019502175 A1 | 08 June 2020 |
| WO 2018081091 A1 | 03 May 2018 | US 2020062760 A1 | 27 February 2020 |
| | | TW 201819386 A | 01 June 2018 |
| WO 2018136265 A1 | 26 July 2018 | TW 201827435 A | 01 August 2018 |
| | | CA 3051206 A1 | 26 July 2018 |
| | | BR 112019015075 A2 | 10 March 2020 |
| | | AU 2018210770 A1 | 15 August 2019 |
| | | JP 2020506178 A | 27 February 2020 |
| | | MX 2019008695 A | 11 September 2019 |
| | | AR 110740 A1 | 02 May 2019 |
| | | US 2020017511 A1 | 16 January 2020 |
| | | IN 201917029275 A | 04 October 2019 |
| | | EP 3571199 A1 | 27 November 2019 |
| | | SG 11201906209 A1 | 27 August 2019 |
| | | KR 20190111079 A | 01 October 2019 |
| | | CN 110446709 A | 12 November 2019 |
| CN 109311848 A | 05 February 2019 | EP 3464272 A4 | 24 April 2019 |
| | | CA 3026784 A1 | 14 December 2017 |
| | | VN 62718 A | 25 April 2019 |
| | | MX 2018015297 A | 12 August 2019 |
| | | WO 2017211303 A1 | 14 December 2017 |
| | | SG 11201810983 A1 | 30 January 2019 |
| | | EP 3464272 A1 | 10 April 2019 |
| | | US 2019127378 A1 | 02 May 2019 |
| | | AU 2017276457 A1 | 24 January 2019 |
| | | JP 2019521181 A | 25 July 2019 |
| | | ID 201902366 A | 05 April 2019 |
| | | PH 12018550202 A1 | 21 October 2019 |

Form PCT/ISA/210 (patent family annex) (January 2015)

**INTERNATIONAL SEARCH REPORT**
**Information on patent family members**

International application No.
**PCT/CN2020/098474**

| Patent document cited in search report | Publication date (day/month/year) | Patent family member(s) | | | Publication date (day/month/year) |
|---|---|---|---|---|---|
| | | KR | 20190015756 | A | 14 February 2019 |
| | | IN | 201917000542 | A | 15 March 2019 |
| | | AU | 2017276457B | B2 | 03 October 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018136264 A
- WO 2015003094 A
- WO 2018160731 A
- WO 2018130928 A1
- WO 2018136265 A
- WO 2018172984 A
- WO 2018081091 A
- WO 2016203405 A
- WO 2017211303 A
- WO 2018013597 A
- WO 2015107495 A1


### Non-patent literature cited in the description


- **ELIEL, E.L. ; WILEN, S. H.** Stereochemistry of Organic Compounds. John Wiley and Sons, Inc, 1994
- *J. Med. Chem.,* 2016, vol. 59, 7773-7782